(19) Europäisches Patentamt
European Patent Office
Office européen des brevets

(11) EP 1 573 337 B1

(12) **FASCICULE DE BREVET EUROPEEN**

(45) Date de publication et mention de la délivrance du brevet:
**14.02.2007 Bulletin 2007/07**

(21) Numéro de dépôt: **03799682.4**

(22) Date de dépôt: **19.12.2003**

(51) Int Cl.:
***G01N 33/68*** *(2006.01)* ***C12Q 1/37*** *(2006.01)*

(86) Numéro de dépôt international:
**PCT/FR2003/003857**

(87) Numéro de publication internationale:
**WO 2004/059322 (15.07.2004 Gazette 2004/29)**

(54) **PROCEDE DE DETECTION DE LA PRP UTILISANT UN LIGAND ADJUVANT MACROCYCLIQUE.**

VERFAHREN ZUR DETEKTION VON PRP UNTER VERWENDUNG EIN MAKROZYKLISCHEN LIGAND

PRP DETECTION METHOD USING AN ANCILLARY MACROCYCLIC LIGAND

(84) Etats contractants désignés:
**AT BE BG CH CY CZ DE DK EE ES FI FR GB GR HU IE IT LI LU MC NL PT RO SE SI SK TR**

(30) Priorité: **20.12.2002 FR 0216383**

(43) Date de publication de la demande:
**14.09.2005 Bulletin 2005/37**

(73) Titulaires:
- **Agence Française de Securité Sanitaire des Aliments**
**94701 Maisons-Alfort Cedex (FR)**
- **CENTRE NATIONAL DE LA RECHERCHE SCIENTIFIQUE (CNRS)**
**75794 Paris Cedex 16 (FR)**
- **UNIVERSITE CLAUDE BERNARD - LYON 1**
**F-69622 Villeurbanne Cédex (FR)**
- **BIOMERIEUX**
**69280 Marcy-L'Etoile (FR)**

(72) Inventeurs:
- **MOUSSA, Aly**
**F-69600 Oullins (FR)**
- **COLEMAN, Anthony, William**
**F-69300 Calluire-Et-Cuire (FR)**
- **SHAHGALDIAN, Patrick**
**F-69003 Lyon (FR)**
- **DA SILVA, Eric**
**F-69800 Saint Priest (FR)**
- **MARTIN, Ambroise**
**F-69390 Charly (FR)**
- **LAZAR, Adina Nicoleta**
**69100 Villeurbanne (FR)**
- **LECLERE, Edwige**
**F-38000 Grenoble (FR)**
- **DUPIN, Marilyne**
**F-69670 Vaugneray (FR)**
- **PERRON, Hervé**
**F-69290 Saint-Genies-Les-Ollières (FR)**

(74) Mandataire: **Breese Derambure Majerowicz**
**38, avenue de l'Opéra**
**75002 Paris (FR)**

(56) Documents cités:
**WO-A-01/23425**
**WO-A-97/37995**
**US-A- 5 571 911**
**WO-A-01/77687**
**WO-A-02/086511**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 573 337 B1

Printed by Jouve, 75001 PARIS (FR)

## Description

La présente invention concerne un procédé de détection des formes du prion pathogènes responsables des encéphalopathies spongiformes subaiguës transmissibles.

La protéine prion native ou normale, désignée PrP ou $PrP^c$ pour la protéine prion cellulaire, est une glycoprotéine largement exprimée dans les cellules lymphoides et neuronales des mammifères.

Des changements conformationnels de $prp^c$ conduisent à l'apparition et à la propagation de la protéine pathogène $PrP^{sc}$ qui est résistante à la protéinase K. Cette protéine pathogène peut être appelée $PrP^{sc}$ ou $PrP^{res}$ indifféremment. L'accumulation de $PrP^{sc}$ dans les organes des animaux est à l'origine de nombreuses maladies et notamment de la tremblante des petits ruminants, de la maladie cachectisante chronique (ou chronic wasting disease 'CWD') de l'élan et de l'antilope, de l'encéphalopathie spongiforme bovine (ESB) et de la maladie de Creutzfeld-Jakob (MCJ) chez l'homme.

L'apparition tardive après une période d'incubation de 2 à 6 ans et le lent développement des symptômes chez le bétail infecté par l'ESB a considérablement ralenti le développement de modèles épidémiologiques. L'ESB est transmissible par ingestion à l'homme et a conduit à l'apparition d'une nouvelle forme de la maladie de Creutzfeld-Jakob (vMJC).

La détection de la protéine pathogène $Prp^{sc}$ est difficile chez les animaux infectés sains avant le développement de la maladie et surtout dans le sang et l'urine chez les animaux malades. Il est à présent établi que la $prp^{sc}$ présente chez les animaux destinés à l'alimentation humaine se transmet à l'homme lors de l'ingestion des tissus infectés. Un objectif majeur de santé publique est donc d'éviter cette transmission en détectant la présence de la $prp^{sc}$ :

- chez des animaux destinés à la consommation humaine en vue de les retirer de la chaîne alimentaire,
- dans les dons de sang et dérivés sanguins destinés à la transfusion chez l'homme. En effet, comme le montre une présence de protéine pathogène $PrP^{sc}$ dans le sang et les liquides lymphoïdes bien avant l'atteinte cérébrale, et donc bien avant la possibilité de déceler des signes neurologiques évocateurs de maladie à prions cliniquement déclarée, la physiopathologie chez l'homme est méconnue et, ne pouvant réaliser des infections expérimentales comme chez le mouton, l'absence de test de détection dans le sang ou d'autres fluides biologiques ne permet pas de l'étudier et donc de prévenir une transmission interhumaine par don de sang, ou de traiter les personnes infectées avant que les lésions cérébrales n'aient débuté ; et
- dans les troupeaux animaux avant le stade neurologique, permettant ainsi d'éliminer les animaux infectés précocement, avant arrivée aux abattoirs.

La détection de la présence de $PrP^{sc}$ dans des échantillons biologiques ou chez des animaux devient donc d'une extrême importance et plusieurs équipes de chercheurs développent des méthodes de détections immunologiques (WO02/086511). Par ailleurs, des méthodes pour complexer à la $prp^{sc}$ des peptides, des petites molécules ou des inhibiteurs en vue du traitement de vMJC font l'objet de recherches actives. Toutefois, les méthodes en l'état de la technique se heurtent sans arrêt à la difficulté d'identifier la $prp^{sc}$ de manière fiable lorsqu'elle est en faible quantité dans un échantillon biologique, et notamment dans les fluides biologiques.

La présente invention a donc pour objet un procédé permettant de détecter la protéine PrP et notamment $prp^{sc}$ à des dilutions où elle n'est pas détectable avec les méthodes utilisées actuellement, cette détection étant d'une fiabilité de 100 % sur les 119 échantillons testés par les inventeurs. Avantageusement, le procédé selon l'invention permet d'amplifier d'un facteur 4 la détection de la $Prp^{sc}$.

Plus précisément, la présente invention a pour objet un procédé de détection de la PrP, et en particulier de la $Prp^{sc}$, caractérisé en ce que l'on ajoute dans un échantillon biologique susceptible de contenir de la PrP, un ligand adjuvant macrocyclique (AML) libre ou lié à un support, puis on fait réagir la suspension résultante avec un anticorps anti-PrP, puis on identifie la présence de PrP.

La liaison du ligand macrocyclique au support peut être mise en oeuvre de façon connue de l'homme du métier, telle que par adsorption ou liaison covalente, la liaison covalente, autrement appelée greffage, étant préférée.

Lorsqu'on cherche à détecter la $Prp^{sc}$, la présente invention peut également comprendre l'étape supplémentaire de traitement de l'échantillon avec de la protéinase K avant ou après contact avec l'AML.

La présente de PrP peut être mise en oeuvre par détection de la réaction anticorps anti-PrP/PrP, et ce par tout moyen connu de l'homme du métier, et notamment par méthode sandwich telle que ELISA, immunoblotting, immuno micro-cantilever détection, spectrométrie de masse ou par des analyses optiques et spectroscopiques. L'identification du complexe AML-PrP s'effectue au moyen de techniques de balayage profilométrique telles que la microscopie à réflectance de balayage, la microscopie de balayage en champ proche ou la microscopie de balayage confocale.

Selon un mode de réalisation préféré de l'invention, l'échantillon biologique provient d'un animal, y compris l'homme. De préférence, cet échantillon provient du cerveau, des tissus du système nerveux central, ou des organes, et notamment la rate ou l'intestin. Suivant un mode de réalisation préféré de l'invention, cet échantillon est un fluide

biologique, notamment le liquide céphalo-rachidien ou le sérum.

Par ligand macrocyclique, on entend un composé capable de se lier à la PrP, et en particulier à la PrPsc et qui est constitué d'une succession de cycles formant un macrocycle. Les ligands macrocycliques sont connus de l'homme du métier. A titre d'exemples non limitatifs, on peut citer les cyclophanes, les métacyclophanes, les cyclodextrines, les cyclo(tétra-acides-chromotropiques), les sphérands et les cyclo[n]vératrylènes.

Avantageusement, le ligand adjuvant macrocyclique est capable de se lier à des sites contigus, ou proches dans l'espace, de sites de liaison avec un anticorps et a pour action d'augmenter la liaison anticorps-$PrP^{sc}$.

Avantageusement, le ligand adjuvant macrocyclique est choisi dans la famille des métacyclophanes. Parmi les métacyclophanes, les para-sulfonato-calix-arènes fonctionnalisés sur la face phénolique sont particulièrement avantageux. Ces composés peuvent être obtenus suivant la méthodologie décrite dans Da Silva et al., J. Supramol. Chem 1, (2001), 135-138.

Avantageusement, le ligand adjuvant macrocyclique répond à la formule générale (I) ci-dessous,

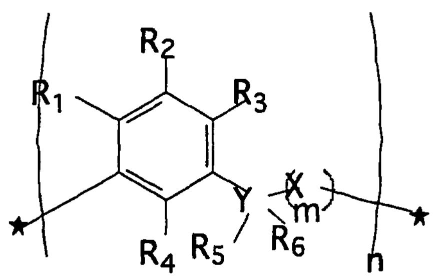

(I)

dans laquelle

- $R_1$ représente un atome d'hydrogène, un groupe hydroxyle, un groupe OR ou un groupe OCOR, R étant tel que défini ci-dessous,
- $R_2$ représente un atome d'hydrogène, un groupe R, COR, Pol, $CH_2Pol$, dans lesquels Pol représente un groupe phosphate, sulfate, amine, ammonium, acide carboxylique et R est tel que défini ci-dessous,
- $R_3$ représente un atome d'hydrogène, un groupe hydroxyle, un groupe OR ou un groupe OCOR dans lesquels R est tel que défini ci-dessous,
- $R_4$ représente un atome d'hydrogène, un groupe hydroxyle, un groupe OR, un groupe $OCH_2R$ ou un groupe OCOR, dans lesquels R est tel que défini ci-dessous,
- Y est un atome de carbone, d'azote, ou un atome de soufre,
- $R_5$ et $R_6$, chacun indépendamment, sont absents ou représentent un atome d'hydrogène, un groupement $CH_2$ ou R tel que défini ci-dessous, ou bien
- $R_5$ et $R_6$ représentent ensemble un atome d'oxygène ou de soufre,
- X représente un groupement $CH_2$ ou un atome d'oxygène ou de soufre,
- m représente un entier égal à 0 ou 1,
- R représente un atome d'hydrogène ou une chaîne hydrocarbonée, saturée ou non, ramifiée ou non, cyclique ou non cyclique, substituée ou non par un groupement halogène, et portant des fonctions polaires ou non polaires,
- n est un entier compris entre 3 et 15,

les substituants $R_1$ à $R_5$, R, X, Y et l'entier m peuvent être de nature différente suivant les motifs.

Ainsi, le composé de formule (I) se présente sous forme d'une succession de n motifs caractérisés par la présence d'un cycle benzénique, et les substituants de ce cycle peuvent être variables d'un motif à l'autre, dans la limite de leurs définitions ci-dessus.

Les ligands macrocycliques peuvent être préparés selon les techniques connues de l'homme du métier, par exemple décrites dans Comprehensive Supramolecular Chemistry, Pergamon, Oxford, 1996.

Les chaînes hydrocarbonées, saturées ou non, ramifiées ou non, cycliques ou non cycliques, substituées ou non par un groupement halogène, et portant des fonctions polaires ou non polaires, sont largement connues de l'homme du métier. A titre d'exemples, on peut citer les alkyles, les alcènes, les aryles et les cycles saturés tels que le cyclohexane. Un exemple de groupement non polaire est $CF_3$ et des exemples de groupements polaires sont les substituants Pol tels que définis précédemment.

Suivant un mode de réalisation particulier de l'invention, le ligand adjuvant macrocyclique répond à la formule

générale (I) ci-dessus, et plus précisément à la formule générale (Ia) ci-dessous

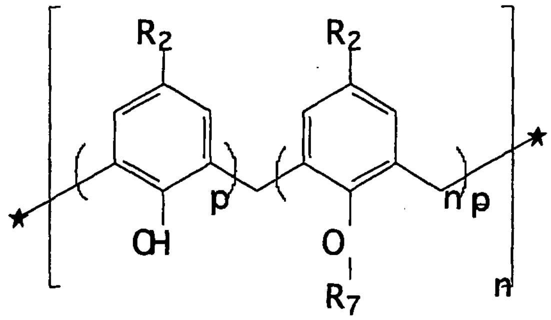

(Ia)

dans laquelle

chaque groupement $R_2$, pris indépendamment, est un groupement sulfate ou un groupement phosphate,
$R_7$ est un groupement $(CH_2)_r$-Z dans lequel Z est un groupement COOEt, COOH, CN ou $NH_2$,
r est un entier compris entre 1 et 20,
p est un entier compris entre 1 et 10,
n est un entier compris entre 2 et 10.

Suivant un mode de réalisation préféré de l'invention, le ligand adjuvant macrocyclique est le p-sulphonato-calix-[4]-arène, le p-sulphonato-calix-[6]-arène, le p-sulphonatocalix-[8]-arène, ou l'un de leurs dérivés.
Suivant un second mode de réalisation de l'invention, le ligand adjuvant macrocyclique répond à la formule générale (I) ci-dessus, et plus précisément à la formule générale (Ib) ci-dessous

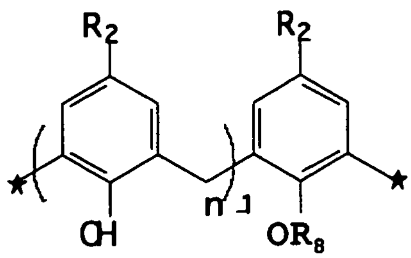

(Ib)

dans laquelle n est un entier compris entre 4 et 8,
chaque groupement $R_2$, pris indépendamment, est un groupement sulfate ou un groupement phosphate,
$R_8$ représente un groupement $(CH_2)_t$-$(CO)_s$-$(NH_2)$ ou un groupement $(CH_2)_t$-COOH où t est un entier compris entre 0 et 6 et s est un entier compris entre 0 et 6.

Avantageusement, le calix-arène de formule (Ib) est tel que les deux groupements $R_2$ sont chacun un groupement sulfate, n est 4, 6 ou 8, et $R_8$ est un atome d'hydrogène, un groupement $CH_2COOH$, un groupement $CH_2CONH_2$ ou un groupement $CH_2CH_2NH_2$.
De préférence, le calix-arène de formule (Ib) est tel que tel que les deux groupements $R_2$ sont chacun un groupement sulfate, n est un entier égal à 6, et $R_8$ est un groupement $(CH_2)_2$-$NH_2$.
Suivant un mode de réalisation particulier de l'invention, le ligand selon l'invention est greffé sur un support solide. Ce support est fonctionnalisé par une fonction susceptible de former une liaison avec une fonction portée par le ligand. Suivant un mode de réalisation préféré, le support solide est fonctionnalisé par une liaison NHS (N-hydroxysuccinimide) ou par une fonction $NE_2$. Cette fonction peut réagir avec une fonction portée sur le ligand. Dans ce mode de réalisation, les calix-arènes portant une fonction susceptible de réagir pour former une liaison avec la liaison fonctionnelle du support solide, notamment les calix-arènes portant une liaison $NH_2$ ou COOH, sont particulièrement préférés.
L'invention a également pour objet un ligand adjuvant macrocyclique greffé sur un support fonctionnalisé, dans lequel le ligand répond à la formule (Ib) telle que définie ci-dessus, dans laquelle n est un entier compris entre 4 et 8,

et le support est du type support solide de préférence fonctionnalisé par un groupe NHS ou un groupe $NH_2$ et est de préférence une bille magnétique ou une microplaque. Un tel greffage préserve l'interaction ultérieure entre la protéine et le ligand, ainsi que la présentation stéréochimique de l'épitote pour la détection par un anticorps anti-PrP, les sites ciblés par le greffage sur le support étant différents de ceux qui servent à l'interaction avec la PrP.

Selon un autre mode de réalisation de l'invention, ledit ligand adjuvant macrocyclique est choisi dans la famille des cyclodextrines et en particulier des cyclodextrines répondant à la formule générale (II)

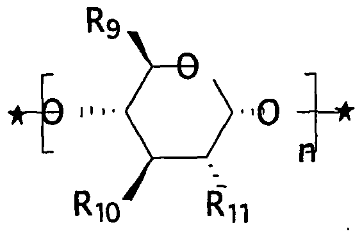

(II)

dans laquelle

$R_9$ représente un atome d'hydrogène ou un groupement $CH_2$, OH, OR, OCOR, COR, $CH_2$Pol, $OCH_2R$, SR, NR, Pol et R étant ci-dessous définis,

$R_{10}$ et $R_{11}$, chacun indépendamment, représentent un atome d'hydrogène ou un groupement $CH_2$, OH, OR, OCOR, COR, $CH_2$Pol, $OCH_2R$, Pol et R étant ci-dessous définis,

Pol représente un groupe phosphate, sulfate, amine, ammonium ou acide carboxylique,

R représente une chaîne hydrocarbonée, saturée ou non, ramifiée ou non, cyclique ou non cyclique, substituée ou non par un groupement halogène, et portant des fonctions polaires ou non polaires,

n est un entier compris entre 6 et 8,

les substituants $R_9$ à $R_{11}$ peuvent être de nature différente suivant les motifs. Ainsi, le composé de formule (II) se présente sous forme d'une succession de n motifs caractérisés par la présence d'un cycle, et les substituants de ce cycle peut être variables d'un motif à l'autre, dans la limite de leurs définitions ci-dessus.

Selon une variante de réalisation de l'invention, ledit ligand adjuvant macrocyclique est choisi dans la famille des cyclo-tétra-acides chromotropiques, et en particulier aux cyclo-tétra-acides chromotropiques répondant à la formule générale (III)

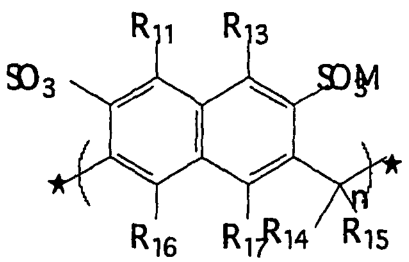

(III)

dans laquelle

$R_{12}$ et $R_{13}$, chacun indépendamment, représentent un atome d'hydrogène ou un groupe R ou Pol tels que définis ci-dessous,

$R_{14}$ et $R_{15}$, chacun indépendamment, représentent un atome d'hydrogène, un groupement $CH_2$ ou un groupe R tel que défini ci-dessous, ou bien

$R_{14}$ et $R_{15}$ représentent ensemble un atome d'oxygène ou de soufre,

$R_{16}$ et $R_{17}$, chacun indépendamment, représentent un atome d'hydrogène ou un groupe hydroxyle ou un groupe OR, $OCH_2R$, OCOR, SR, $SCH_2R$, SCOR, R étant tel que défini ci-dessous,

M représente un atome d'hydrogène, ou un atome choisi parmi Na, K, Li, Cs, Rb, Mg et Ca,

Pol représente un groupe phosphate, sulfate, amine, ammonium ou acide carboxylique,

R représente une chaîne hydrocarbonée, saturée ou non, ramifiée ou non, cyclique ou non cyclique, substituée ou

non par un groupement halogène, et portant des fonctions polaires ou non polaires,
n est un entier compris entre 3 et 15,

les substituants $R_{12}$ à $R_{17}$, M, Pol, et R peuvent être de nature différente suivant les motifs. Ainsi, le composé de formule (III) se présente sous forme d'une succession de n motifs caractérisés par la présence d'un cycle, et les substituants de ce cycle peuvent être variables d'un motif à l'autre, dans la limite de leurs définitions ci-dessus.

Selon un autre mode de réalisation de l'invention, ledit ligand adjuvant macrocyclique est choisi dans la famille des cyclo[n]veratrylenes, répondant à la formule générale (IV)

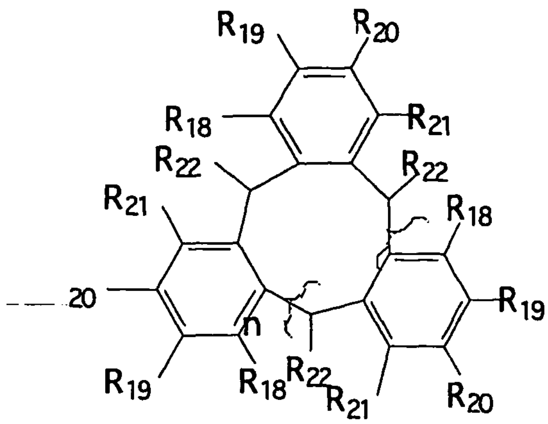

(IV)

dans laquelle

$R_{18}$ représente un atome d'hydrogène ou un groupement $CH_2$, OH, OR, OCOR, COR, $CH_2$pol, $OCH_2R$, SR, NR, Pol et R étant ci-dessous définis,
$R_{19}$, $R_{20}$, $R_{21}$ et $R_{22}$, chacun indépendamment, représentent un atome d'hydrogène ou un groupement $CH_2$, OH, OR, OCOR, COR, $CH_2$Pol, $OCH_2R$, Pol et R étant ci-dessous définis,
Pol représente un groupe phosphate, sulfate, amine, ammonium ou acide carboxylique,
R représente une chaîne hydrocarbonée, saturée ou non, ramifiée ou non, cyclique ou non cyclique, substituée ou non par un groupement halogène, et portant des fonctions polaires ou non polaires,
n est un entier compris entre 1 et 10,

les substituants $R_{18}$ à $R_{22}$, R, Pol et R peuvent être de nature différente suivant les motifs. Ainsi, le composé de formule (IV) se présente sous forme d'une succession de n motifs caractérisés par la présence d'un cycle, et les substituants de ce cycle peuvent être variables d'un motif à l'autre, dans la limite de leurs définitions ci-dessus.

Un autre objet de l'invention réside dans l'utilisation d'un ligand adjuvant macrocyclique (AML) pour la détection du prion $PrP^{sc}$ dans un échantillon biologique, et en particulier d'un ligand de formule (I), (Ia) ou (Ib), ci-dessus, libre ou lié à un support.

L'invention a également pour objet un kit de diagnostic des maladies dont la $prp^{sc}$ est responsable, du type ESB, la tremblante des petits ruminants, Creutzfeld-Jakob, caractérisé en ce qu'il comprend l'utilisation du ligand adjuvant macrocyclique et en particulier d'un ligand de formule (I), (Ia) ou (Ib) ci-dessus, libre ou lié à un support.

L'invention a également pour objet un kit de dosage immunologique de la $prp^{sc}$ caractérisé en ce qu'il comprend l'utilisation d'un ligand adjuvant macrocyclique et en particulier d'un ligand de formule (I), (Ia) ou (Ib) ci-dessus, libre ou lié à un support.

D'autres avantages et caractéristiques de l'invention apparaîtront à la lecture des exemples qui suivent concernant l'amplification de la détection de la $PrP^{sc}$ lorsque l'échantillon biologique est mis en présence d'un ligand adjuvant macrocyclique, libre ou greffé.

Il sera fait référence dans ces exemples aux dessins en annexe dans lesquels :

En référence à l'exemple 1 :

- la figure 1 est un exemple comparatif de la détection par immunoblotting de $prp^{sc}$ d'une part dans un échantillon mis en présence d'un ligand adjuvant macrocyclique spécifique (AML1) dont la préparation est décrite ci-après et, d'autre part, dans un échantillon non mis en présence d'AML1;

- la figure 2 représente les courbes de densité optique de détection de $PrP^{sc}$ d'une part dans un échantillon mis en présence d'AML1 et, d'autre part, dans un échantillon non mis en présence d'AML1 ;
- la figure 3 montre des images en microscopie de sonde à balayage (SPM) en mode non-contact pour des films séchés de la protéine prion recombinante (recPrP) seule (A), de recPrP en présence d'AML1 (B) et d'AML1 seul (C) ;
- la figure 4 montre des images en microscopie de sonde à balayage (SPM) en mode non-contact pour des films séchés de recPrP en présence d'AML dans différentes proportions : 1/1000 AML1/recPrP, 1/100 AML1/recPrP, 1/10 AML1/recPrP.
- la figure 5 est une représentation graphique donnant les valeurs de densité optique (DO) obtenues en fonction de la concentration en calix-arène C6S greffé sur une plaque amine activée (NHS) après incubation d'une solution de PrP recombinante bovine et révélation par l'anticorps anti-PrP AC23 marqué à la peroxydase,
- la figure 6 est une représentation graphique donnant les valeurs de densité optique (DO) obtenues en fonction de la concentration en calix-arène C6S greffé sur une bille amine activée (NHS) après incubation d'une solution de PrP recombinante humaine dosée à 0,25 µg/ml par révélation directe avec l'anticorps anti-PrP 8D11G12 marqué à la peroxydase,
- la figure 7 est une représentation graphique donnant les valeurs de densité optique (DO) obtenues après incubation avec une gamme de dilution de sérum humain dans du PBST 0,05% mise en contact avec du calix-arène C6S greffé sur une bille amine activée (NHS), par révélation directe avec l'anticorps anti-PrP 8D11G12 marqué à la peroxydase,
- la figure 8 est une représentation graphique donnant les valeurs de densité optique (DO) obtenues après mise en contact de calix-arène C6S greffé sur une plaque amine activée (NHS) avec des échantillons de liquide céphalo-rachidien humains positifs (LCR+) et négatifs (LCR-) pour la MJC en fonction du chauffage, par révélation avec l'anticorps anti-PrP AC23 marqué à la phosphatase alcaline,
- la figure 9 est une représentation graphique donnant les valeurs de densité optique (DO) obtenues après mise en contact de calix-arène C6S greffé sur une plaque amine activée (NHS) avec de la $prp^{sc}$ extraite de cerveaux de patients atteints ou non de la maladie de Creutzfeld-Jakob (MJC+/MJC-), par révélation avec l'anticorps anti-PrP AC23 marqué à la phosphatase alcaline,
- la figure 10 est une représentation graphique donnant les valeurs de densité optique (DO) obtenues en fonction de la concentration de calix-arène C6S greffé sur une plaque amine activée (NHS) après mise en contact avec un échantillon de LCR de patient non atteint de MJC, dilué au 1/2 ou au 1/6, et en l'absence de digestion par la protéinase K,
- la figure 11 est une représentation graphique donnant les valeurs de densité optique (DO) obtenues en fonction de la concentration de calix-arène C6S greffé sur une plaque amine activée (NHS) après mise en contact avec un échantillon de LCR de patients non atteints de MJC (LCR-) ou atteints de cette maladie (LCR+), éventuellement dilué au 1/2 (LCR+1/2 ou LCR-1/2), en l'absence de digestion par la protéinase K, et

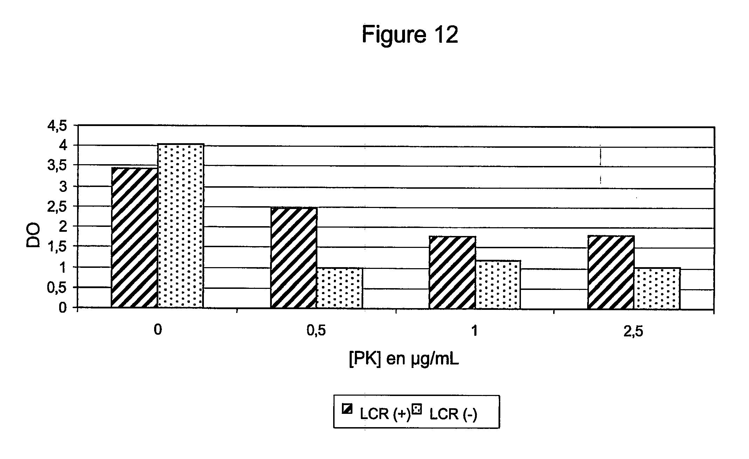

- la figure 12 est une représentation graphique donnant les valeurs de densité optique (DO) obtenues en fonction de la concentration en protéinase K après mise en contact de calix-arène C6S greffé sur une plaque amine activée (NHS) avec un échantillon de LCR de patients non atteints de MJC (LCR-) ou atteints de cette maladie (LCR+),

**Exemple 1 : Préparation des ligands adjuvants macrocycliques**

1.1 Préparation du *p*-sulphonato-3,7-(2-carboxy-methyloxy)-calix-[6]-arène (dénommé AML 1)

Ce ligand adjuvant macrocyclique AML1 possède la formule générale (V) :

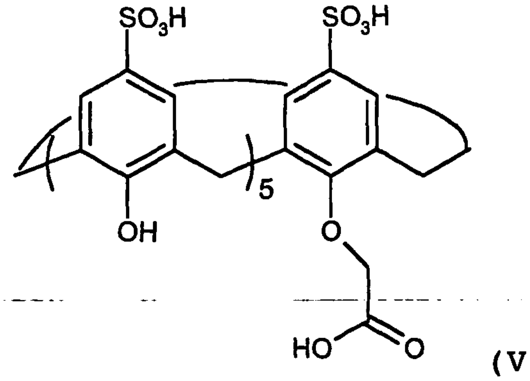

(V)

Ce ligand est synthétisé comme suit : une suspension de calix[6]arène dans l'acétonitrile en présence d'un équivalent de base ($K_2CO_3$) est agitée à reflux. Après 30 minutes, 0,5 équivalent d'agent alkylant (bromo-acétate d'éthyle) sont ajoutés dans le mélange réactionnel qui est maintenu à reflux et sous agitation pendant 24 heures. Après refroidissement de la suspension, le solvant est évaporé sous pression réduite et le solide résultant est dissous dans 200 mL de $CH_2Cl_2$ et lavé deux fois avec une solution d'acide chlorhydrique (1M) et une fois avec de l'eau déminéralisée. Après avoir été séché sur sulfate de magnésium ($MgSO_4$) le solvant organique est évaporé sous pression réduite et permet l'obtention d'une poudre blanche. Celle-ci est purifiée sur une colonne de chromatographie de silice (éluant : $CH_2Cl_2$:hexane; 4 :1). Le produit purifié est analysé en RMN du $^1H$, $^{13}C$ et en spectrométrie de masse electrospray. La saponification de l'ester carboxylique est réalisée par l'hydroxyde de potassium en solution dans un mélange eau : éthanol (30:70) sous agitation à température ambiante pendant 24 heures. Le précipité formé est filtré puis lavé avec 50 mL d'acide chlorhydrique (1M) et 50 mL d'eau, puis purifié sur colonne de chromatographie de silice (éluant chloroforme : méthanol : acide acétique ; 95 :5 : 0.1%) et permet d'obtenir, après évaporation du solvant, un solide blanc et cristallin. Ce produit est analysé en RMN du $^1H$, $^{13}C$ et en spectrométrie de masse electrospray. La sulfonation est réalisée par un traitement avec l'acide sulfurique à 50°C pendant 24 heures. Le produit est précipité avec de l'éther et permet d'obtenir l'AML1 sous forme d'une poudre blanche cristalline.

1.2 Préparation du *p*-sulphonato-3,7-(2-amino-éthyloxy)-calix-[6]-arène (dénommé C6S)

Ce ligand adjuvant macrocyclique C6S possède la formule générale (VI) :

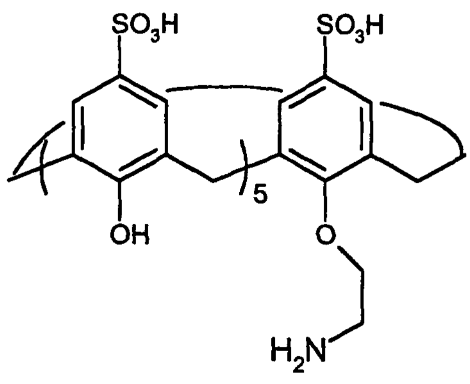

(VI)

Ce ligand est préparé selon la méthode décrite dans Eric Da Silva et Anthony W. Coleman, Synthesis and complexation properties towards amino acids of mono-substituted p-sulphonato-calix-[n]-arene, Tetrahedron 59 (2003) 7357-7364.

1.3 Greffage du Ligand C6S sur plaques et sur billes

Ce ligand est couplé avec un support solide (bille ou plaque) portant une surface activée.

Greffage sur plaque :

Les plaques « NHS activated plates » de 96 puits proviennent de la société Covalab (Lyon, France). 100µL d'une solution de ligand sont dissous à différentes concentrations dans du Tampon Phosphate 50mM pH 8,2. Les puits sont lavés 3 fois (3 x 200µL) à l'eau MilliQ après deux heures d'incubation à 37°C. Les plaques sont séchées à température ambiante avant leur utilisation.

Schéma de greffage :

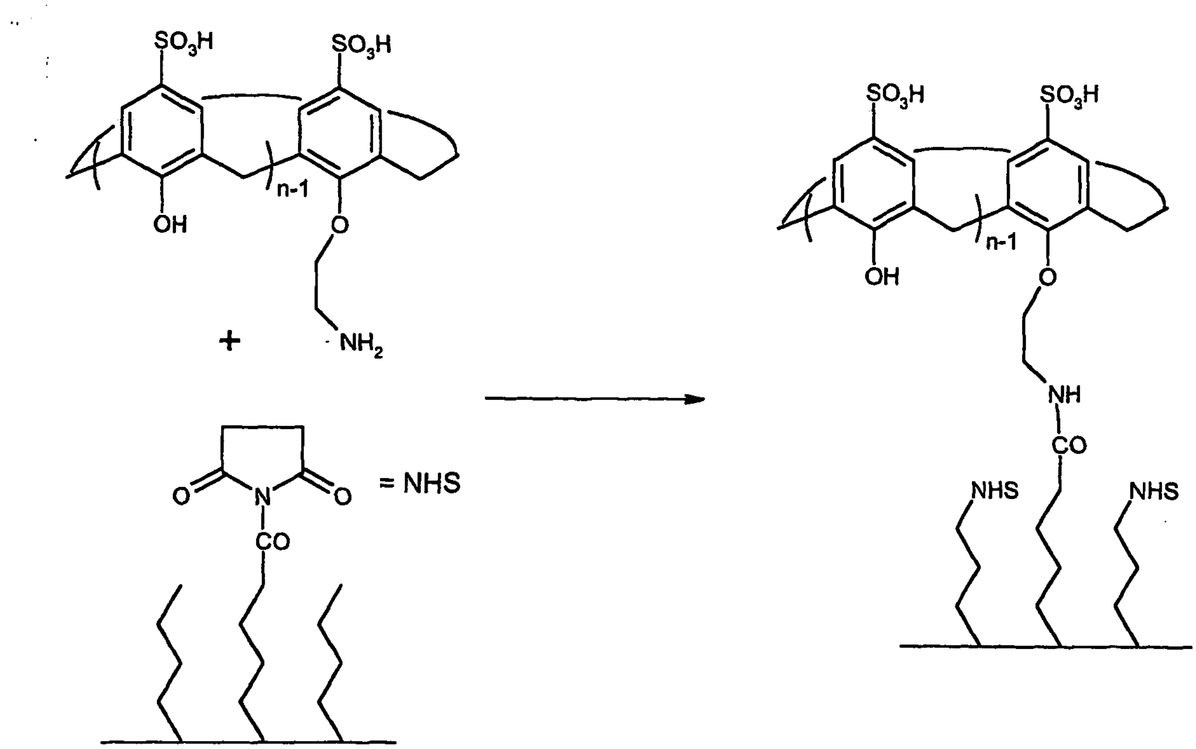

Plusieurs plaques ont été faites à différentes concentrations en ligand C6S. Ces plaques sont définies comme « plaques C6S » dans tout le texte.

Greffage sur billes :

4 mL d'une solution de bille activée NHS (2 x $10^9$ billes /mL ; Dynabeads® M270Amine, Société Dynals, Norvège) sont aliquotés en tubes de 1 mL. Les billes sont centrifugées et précipitées par aimantation. Le surnageant est enlevé et les billes sont lavées 3 fois avec 1 mL d'eau. Le culot de bille est repris avec différents volumes de solution de calixarène dans un tampon phosphate 50mM pH 8,2. 600µL, 120 µL, 60 µL et 12 µL d'une solution de ligand à 50 mg/mL (tampon phosphate 50mM, pH 8,2) sont ajoutés. Les billes sont agitées pendant 24 heures à température ambiante. Les billes sont lavées 3 fois avec de l'eau MilliQ 18Ω afin d'éliminer le ligand qui n'a pas réagi. Les billes sont conservées dans 1 mL d'eau afin de reconstituer la concentration initiale de 2 x $10^9$ billes / mL. La solution de bille est prête à l'emploi. Ces billes sont définies comme « billes C6S » dans tout le texte.

Schéma de greffage du C6S sur les billes :

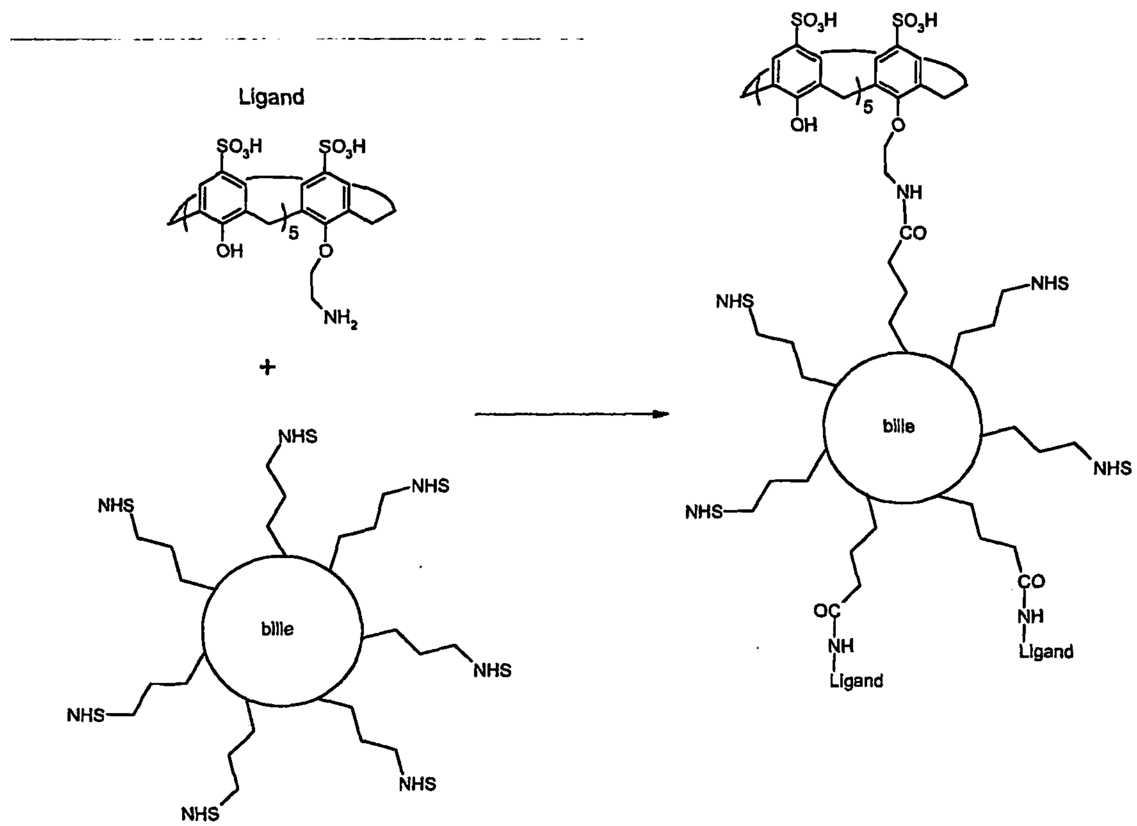

1.4 Greffage de AML1 sur une surface minérale modifiée

1 mL de 3-Aminopropyltri-methoxysilane (Société ABCR) est mis en solution dans une solution de 50mL (95% Ethanol et 5% $H_2O$) pendant 5 min. la plaque de silicium est plongée dans cette solution pendant 4 min sous agitation. On rince la plaque de silicium avec de l'éthanol puis la plaque est mise à sécher à 130°C pendant 15 min. 10µL de AML1 en solution 0,1M dans le DMSO ont été couplés sur une plaque de silicium modifiée en utilisant un agent de couplage (DCC/HOBT). L'échantillon a été séché pendant 12 heures à 23°C.

**Schéma de couplage :**

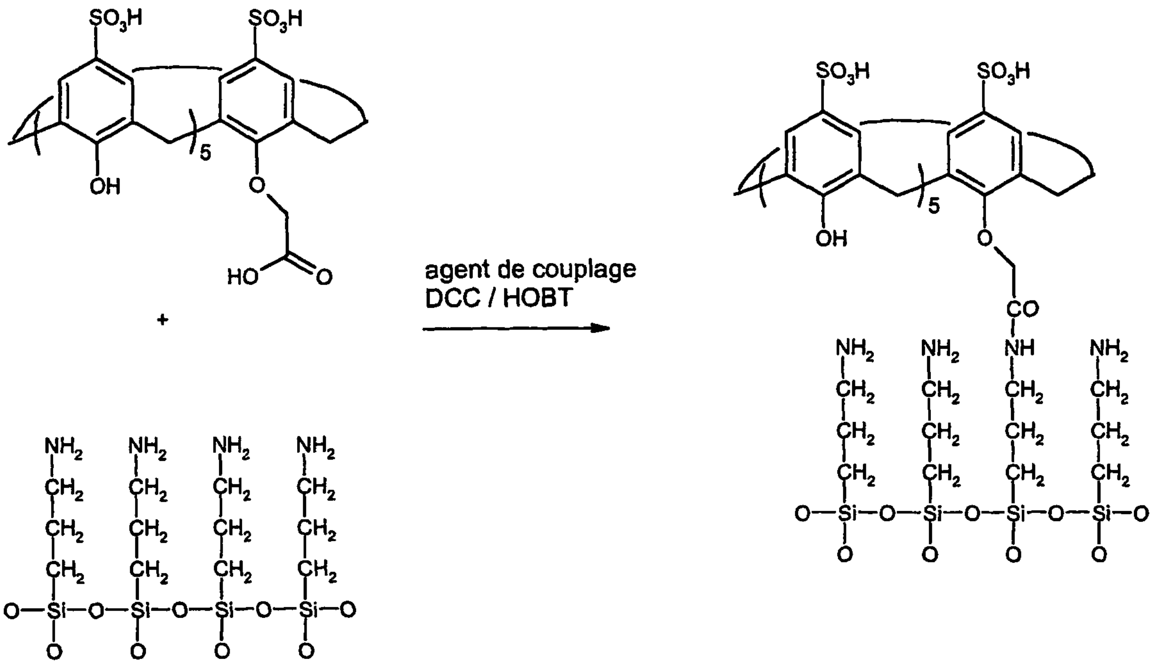


**Exemple 2 : Détection de PrP en utilisant l'AMLI non couplé à un support solide**

2.1 Préparation des échantillons

Les échantillons qui ont servi à la réalisation des exemples des figures 1 et 2 ont été préparés comme suit :

Echantillon sans AML : 0,5g de tissu de cerveau est broyé dans 4,5 mL de solution glucose5 % pour obtenir une suspension à 10% en poids/volume. Pour 100 μl d'homogénat de cerveau à 10 % dans du glucose à 5 % (équivalent à 10 mg de cerveau) est ajoutée 1 μg de protéinase K (Boehringer) dans 10 μl. La solution est mise sous vortex et incubée à 37°C pendant une autre heure. Après avoir ajouté 100 μl de tampon Laemmli dénaturant, on chauffe 5 minutes à 100 °C, on centrifuge à 12000 G pendant 5 mn, et on récupère les surnageants pour les faire migrer sur SDS PAGE.

Echantillon avec AML : 0,5g de tissu de cerveau est broyé dans 4,5 mL de solution glucose 5 % pour obtenir une suspension à 10% en poids/volume. Pour 100 μl d'homogénat de cerveau à 10 % dans du glucose à 5 % (équivalent à 10 mg de cerveau), on ajoute 1 μl d'AML 0,1M. Après une heure à 37°C, 1,5 μg de protéinase K (Boehringer) dans 10 μl est ajoutée. La solution est mise sous vortex et incubée à 37°C pendant une autre heure. Après avoir ajouté 100 μl de tampon Laemmli dénaturant, on chauffe 5 minutes à 100 °C, on centrifuge à 12000 G pendant 5 mn, et on récupère les surnageants pour les faire migrer sur SDS PAGE.

2.2 Procédé de détection par immunoblotting

Après migration sur un gel d'électrophorèse unidimensionnelle de 15% de polyacrylamide en présence de sodium dodécyl sulfate (SDS PAGE) comme décrit par Laemmli, Nature 227 (1970), 680-685, les protéines sont transférées par électrophorèse sur des membranes nitrocellulose et immunoblottées à température ambiante pendant 60 minutes avec un anticorps monoclonal reconnaissant un épitope spécifique constitué des acides aminés 126-160. L'anticorps secondaire (1/5000) est un anticorps de chèvre dirigé contre les chaînes lourdes et légères des immunoglobulines de souris conjuguées à la peroxydase de raifort (IgG H+L).

Les blots sont ensuite lavés et les signaux sont détectés par chimioluminescence soit avec un kit ECL (Amersham) sur des films (Biomex light, Kodak) ou avec un super Signal Ultra (Pierce) et visualisation sur Fluor S. Multimager (BioRad).

La figure 1 est un exemple comparatif qui montre que la détection du prion est amplifiée au moins quatre fois lorsque le procédé de l'invention est mis en oeuvre, par rapport à la détection de la même protéine en absence d'AML1.

En absence d'AML1, pour des dilutions supérieures au 1/8e, aucune $Prp^{sc}$ n'est détectée. En présence d'AML, la détection de la $prp^{sc}$ dans le même échantillon est possible jusqu'à une dilution au 1/32e. Les résultats de la figure 2 montrent la détection.

2.3 Résultats de microscopie

Les échantillons qui ont servi pour les expériences en microscopie de sonde à balayage reportées dans les figures 3 et 4 ont été préparés comme suit :

Echantillons contenant du ligand adjuvant macrocyclique uniquement : des échantillons contenant du ligand adjuvant macrocyclique (50 mM) sont préparés par dépôt sur du mica fraîchement clivé de 10 μl de solution d'AML respectivement et de 10 μl d'eau et sont séchés pendant 24 heures à 37°C.

Echantillons contenant de la protéine PrP recombinante (recPrP) uniquement : des échantillons contenant du recPrP (50 mM) sont préparés par dépôt sur du mica fraîchement coupé de 10 μl de solution de 1/10 (v/v) AML1/recPrP, 1/100 (v/v) AML1/recPrP et 1/1000 (v/v) AML1/recPrP respectivement et de 10 μl d'eau et sont séchés pendant 24 heures à 37°C.

Echantillons comprenant à la fois du ligand adjuvant macrocyclique et du recPrP : 1) des échantillons contenant à la fois du ligand adjuvant macrocyclique et du recPrP sont préparés par dépôt sur du mica fraîchement clivé de 1 μl de solution d'AML et de 1000 μl de recPrP respectivement et sont séchés pendant 24 heures à 37°C ; 2) des échantillons contenant à la fois du ligand adjuvant macrocyclique (AML1) et du recPrP sont préparés par dépôt sur du mica fraîchement coupé de 1 μl de solution d'AML et de 100 μl de recPrP respectivement et sont séchés pendant 24 heures à 37°C ; 3) des échantillons contenant à la fois du ligand adjuvant macrocyclique et du recPrP sont préparés par dépôt sur du mica fraîchement coupé de 1 μl de solution d'AML et de 10 μl de recPrP respectivement et sont séchés pendant 24 heures à 37°C.

On effectue une analyse par imagerie au moyen d'un Thermomicroscope Explorer AFM équipé avec un scanner trépied 100 μm, en mode non-contact, utilisant des fréquences de résonance élevées ($F_0$ = 320 kHz) de cantilever pyramidal avec des sondes silicones à une fréquence de balayage de 1 Hz. Le traitement des images est réalisé avec le logiciel SPMlab 5.1 et présentées non-filtrées.

Il ressort des figures 3 et 4 que les films de recPrP seuls montrent une structure en agrégats circulaires caractéristique. En présence d'AML, les structures observées pour le recPrP sont modifiées séquentiellement, sont fonction croissante de la quantité d'AML et montrent des motifs arrondis et éventuellement cristallite orthogonal. Les images du ligand adjuvant macrocyclique seul (AML1) montrent des structures ressemblantes, mais en plus petites, aux motifs des films recPrP- AML.

D'autres expériences ont été réalisées par microscopie à force atomique.

Une analyse ultérieure par analyse de surface de la rugosité est donnée dans le tableau I. L'utilisation de ces mesures de rugosité peut être étendue à des techniques d'analyses profilométriques.

TABLEAU 1

| | AML seul | AML/ recPrP 1/1000 | AML/ recPrP 1/100 | AML/ recPrP 1/10 | recPrP seul |
|---|---|---|---|---|---|
| Ra (nm) | 48 | 69,6 | 123,9 | 134,4 | 1,11 |
| RMS (nm) | 58,7 | 82,8 | 149 | 163,1 | 1,95 |
| Hauteur moyenne | 217,7 | 233,3 | 408,7 | 469,8 | 5,4 |
| Valeur maximale de hauteur | 448,9 | 484,3 | 894,66 | 1056,9 | 43,17 |

$$R_a = \frac{1}{N}\sum_{i=1}^{N}\left|z_i - \bar{z}\right|$$

(Formule A)

$$RMS = \sqrt{\frac{1}{N}\sum_{i=1}^{N}\langle z_i - z\rangle^2}$$

(Formule B)

$$Avg\,Height = \frac{1}{N}\sum_{i=1}^{N} z_i$$

(Formule C)

Les valeurs de rugosité calculées sont obtenues par le logiciel du Thermomicroscope SPML 5.01. La rugosité moyenne Ra est définie comme la moyenne arithmétique des déviations de hauteur (formule A), la racine moyenne de la rugosité carrée (root mean square roughness RMS) est définie comme la racine carrée de la valeur moyenne des carrés des distances des points à la valeur moyenne d'image (formule B) et la hauteur moyenne de l'échantillon (formule C).

**Exemple 3 : Détection de PrP recombinante en utilisant le C6S greffé à un support solide**

3.1 Détection de PrP recombinante bovine sur plaques

a) protocole

Le protocole employé dans cette expérience est calqué sur celui réalisé dans un ELISA classique.

Selon l'exemple 1, le greffage des plaques amine activées (NHS) est réalisé durant 6 heures avec une gamme de Calix-6-Arènes Sulfonés (C6S) dilué dans une solution de PBS 50 mM (phosphate buffer saline). Les plaques sont ensuite rincées à l'eau distillée avant d'être saturées par une solution de PBST (PBS Tween) (0,05% lait 5% pendant 1 heure à température ambiante. Après rinçage, est déposée une solution de protéine prion (PrP) recombinante bovine diluée à une concentration de 0,25 µg/ml dans du PBST 0,05%. Elle est incubée une heure à température ambiante. La plaque est de nouveau rincée trois fois. Puis elle est incubée avec une solution d'anticorps anti-PrP marqués à la peroxydase diluée à 0,5 µg/ml dans du PBST 0,05% durant une heure à température ambiante. L'anticorps utilisé reconnaît la région définie par les acides aminés 145-154 de la PrP humaine et les régions homologues des PrP animales (AC23). Enfin, après un nouveau cycle de rinçage, est ajouté le révélateur, une solution d'OPD (ortho-phénylènediamine) qui est incubée 10 minutes à température ambiante à l'abri de la lumière. La réaction est stoppée à l'aide d'une solution d'$H_2SO_4$. La lecture du signal obtenu est effectuée à l'aide d'un spectrophotomètre à 495 nm.

b) Résultats

Les résultats sont indiqués sur la figure 5 qui montre que la densité optique (DO) mesurée en ordonnée reflète la quantité de PrP capturée par les C6S et restée accrochée après lavages. Le signal obtenu pour les différents greffages montre que la PrP recombinante bovine est bien capturée par les C6S. La PrP recombinante bovine se fixe sur les C6S greffés sur les plaques NHS.

3.2 Détection de la PrP recombinante humaine sur billes

a) Conditions expérimentales :

Selon l'exemple 1, les billes NHS sont tout d'abord mises en contact avec une solution de C6S diluée dans de l'eau distillée pour greffage du C6S sur les billes. Après cette période, les billes sont lavées à l'eau distillée puis au PBST 0,05%. Une solution de PrP recombinante humaine dosée à 0,25 µg/ml dans du PBST 0,05% est préparée parallèlement. Puis on réalise une gamme de billes C6S par ajout de respectivement : 0 ; 0,1 ; 1 ; 5 et 10µl de la solution de billes pour 100µl de la solution de PrP. L'incubation dure 1 heure à 37°C, puis les billes sont séparées du surnageant par aimantation. La PrP fixée sur les billes est dosée directement à l'aide d'un anticorps marqué selon une méthode sandwich proche de la méthode ELISA.

L'anticorps de révélation marqué à la peroxydase est incubé une heure à 37°C après rinçages des billes par une solution de PBST 0,05%. Les billes sont séparées du surnageant par aimantation avant d'être de nouveau lavées

puis révélées par une solution d'OPD incubée 10 minutes. La réaction est stoppée à l'aide d'une solution d'$H_2SO_4$. L'anticorps de révélation employé est le 8D11G12 (bioMérieux, France.

La lecture du signal obtenu est effectuée à l'aide d'un spectrophotomètre à 495 nm.

b) Résultats

Les résultats de révélations sur bille sont indiqués sur la figure 6 et montrent que les C6S sont correctement greffées sans perte de fonction sur les supports NHS mais surtout gardent leur propriété de capture de la PrP recombinante de différentes espèces et amplifient même le signal obtenu avec les anticorps spécifiques de cette protéine de façon reproductible.

**Exemple 4 : Détection de PrP physiologique en utilisant le C6S greffé à un support solide**

4.1 Sérum humain sur billes

a) Protocole :

Selon l'exemple 1, les billes NHS sont tout d'abord mises en contact avec une solution de C6S diluée dans de l'eau distillée pour le greffage. Après cette période, les billes sont lavées à l'eau distillée puis au PBST 0,05%.

Une gamme de sérum est préparé par dilution dans du PBS. Une solution de PrP recombinante humaine dosée à 0,25 μg/ml dans du PBS est préparée parallèlement ainsi qu'une solution de PBST 0,05% lait 5% qui serviront respectivement de témoin positif et négatif.

Les billes sont ajoutées à raison de 5 μl d'une solution de 2.10$^{e}$9 billes/ml pour 250 μl d'échantillon. L'incubation dure 1 heure à 37°C sous agitation. Les billes sont ensuite séparées du surnageant par aimantation. La PrP fixée sur les billes est dosée directement à l'aide d'un anticorps marqué.

La révélation sur billes utilise un protocole proche de l'ELISA. L'anticorps de révélation marqué à la peroxydase est incubé une heure à 37°C et sous agitation douce après rinçage des billes par une solution de PBST 0,05%. Les billes sont séparées du surnageant par aimantation avant d'être de nouveau lavées puis révélées par une solution d'OPD incubée 10 minutes. La réaction est stoppée à l'aide d'une solution d'$H_2SO_4$. L'anticorps de révélation employé est le 8D11G12.

La lecture du signal obtenu est effectuée à l'aide d'un spectrophotomètre à 495 nm.

b) Résultats

Les résultats sont indiqués sur la figure 7 qui met en évidence que :.

- le témoin négatif dans le lait permet d'évaluer le bruit de fond tandis que le témoin positif dans une solution de PrP 0.25μg/ml permet de vérifier que l'expérience a fonctionné et est interprétable.
- les C6S greffées sur les billes NHS capturent la PrP physiologique présente dans le sérum humain. De plus, la capture est meilleure lorsque la dilution du sérum est augmentée avec un plateau de saturation atteint pour les dilutions au 1/5$^{ème}$ et au 1/10$^{ème}$.

c) Conclusion

De façon surprenante, les C6S greffées sur billes NHS capturent la PrP physiologique présente dans le sérum humain. Les dilutions du sérum permettent d'augmenter le signal obtenu sur les billes.

4.2 Liquide Céphalorachidien (LCR) humain sur plaques

a) Conditions expérimentales :

Les échantillons de LCR sont tout d'abord répartis en tubes de volume équivalents. Deux points de dilution : pur et au 1/2 sont réalisés pour chaque échantillon. Puis ceux-ci sont soumis à trois types de traitement par la chaleur : 30 minutes à 56°C, 15 minutes à 75°C ou 5 minutes à 95°C. Un témoin sans chauffage est réalisé en parallèle pour les échantillons purs.

Selon l'exemple 1, parallèlement, les plaques NHS sont mises en contact durant 6 heures avec une solution de C6S diluée dans une solution de PBS 50 mM pour le greffage.

Les plaques sont ensuite rincées à l'eau distillée puis au PBST 0,05%.

Les échantillons sont alors déposés et incubés une nuit à 2-8°C . La plaque est ensuite rincée six fois. Puis elle est incubée avec une solution d'anticorps anti-PrP marqués à la biotine diluée à 0,5 μg/ml dans du PBST 0,05% durant une heure à température ambiante. L'anticorps utilisé est le AC23.

Après un nouveau cycle de rinçage, est ajoutée une solution de Streptavidine-PAL (Phosphatase Alcaline) qui est incubée 20 minutes à température ambiante. La plaque est une dernière fois rincée et la solution de révélation de PNPP est déposée et incubée 30 minutes à 37°C. La réaction est stoppée à l'aide d'une solution de NaOH 0,4N.

La lecture du signal obtenu est effectuée à l'aide d'un spectrophotomètre à 405 nm contre un filtre à 490 nm.

b) Résultats :

Les résultats sont indiqués sur la figure 8 qui met en évidence que la PrP physiologique peut être dosée dans le LCR.

**Exemple 5 : Détection de $PrP^{sc}$ dans le cerveau en utilisant le C6S greffé à un support solide**

5.1 Détection sur plaque

a) Conditions expérimentales :

La première étape consiste à obtenir de la $PrP^{sc}$ purifiée par extraction à partir de cerveaux de patients atteints ou non de la maladie de Creutzfeld-Jakob (MJC). Un échantillon de 260 mg de chaque cerveau est prélevé puis broyé afin d'obtenir un homogénat 10% en glucose 5%. Le broyat est ensuite filtré à l'aide d'une aiguille. Puis vient une étape de digestion par la protéinase K. Elle est utilisée à la concentration de 20 μg pour 100 mg de tissu à 37°C pendant 1 heure. Ensuite, 650 μl d'une solution de Sarkosyl 30% sont ajoutés. En parallèle, un coussin de saccharose de 400 μl est déposé au fond d'un tube pour ultracentrifugation. L'échantillon est ensuite déposé sur ce coussin. Les tubes sont complétés avant d'être soudés puis ultracentrifugés 2 heures à 20°C à 100 000 rpm. Les surnageants sont éliminés et les parois des tubes sont grossièrement séchées avec du papier absorbant. Les culots sont ensuite repris dans du Tris-Maléate. Les échantillons sont alors chauffés 5 minutes à 95°C puis centrifugés 15 minutes à 12 000 rpm à 20°C. Les échantillons sont conservés à - 80°C jusqu'à leur prochaine utilisation. La réussite de cette première étape a été confirmée par Western Blot.

b) Test sur plaques Calix-6-Sulphonates

Selon l'exemple 1, le greffage des plaques NHS est réalisé durant 6 heures avec une gamme de C6S dilué dans une solution de PBS 50 mM. Elles sont tout d'abord saturées par une solution de PBST 0,05% lait 5% durant 6 heures à température ambiante. Puis elles sont lavées trois fois par une solution de PBST 0,05%. L'échantillon préalablement dilué en tris maléate est déposé sur la plaque et incubé une nuit à 2-8°C. La plaque est ensuite lavée 6 fois en PBST 0,05%. La solution d'anticorps de révélation est incubée 1 heure à température ambiante. Après une nouvelle série de lavage est incubée la streptavidine-PAL durant 20 minutes. La plaque est lavée une dernière fois avant le dépôt de PNPP (para-nitrophénylphosphate) qui est incubé 30 minutes à 37°C. Puis la réaction de révélation est stoppée par ajout de soude.

La lecture de la DO est effectuée à 405 nm contre un filtre à 490 nm à l'aide d'un spectrophotomètre.

c) Résultats

Les résultats sont indiqués sur la figure 9 qui montre que, de façon surprenante, la $PrP^{sc}$ des échantillons issus de patients atteints de la maladie de Creutzfeld-Jakob est détectable par le procédé de l'invention utilisant une capture par des C6S greffées sur plaques NHS et une détection par un anticorps dirigé spécifiquement contre la protéine PrP, ceci étant validé par les témoins positifs PrP recombinante humaine et négatifs PBST 0,05%.

**Exemple 6 : Détection de $PrP^{sc}$ dans le LCR en utilisant le C6S greffé à un support solide**

6.1 Préparation des échantillons

Les échantillons sont constitués de prélèvements de liquide céphalo-rachidien (LCR) de préférence non hémolysés et sans débris cellulaires. Ces prélèvements sont anonymes et sont conservés congelés à -(80°C) dans des tubes à parois faiblement adsorptives pour les protéines (« Pre-lubricated micro centrifuge tube 1,7 mL », Marsh Biomédical Products, réf. T6050G).

Ils se répartissent en deux catégories :

D'une part, les échantillons positifs pour la MJC.
Ce sont des LCR post-mortem, récupérés lors de l'autopsie par ponction dans les citernes, et dont la recherche de la $PrP^{sc}$ dans le tissu cérébral par western blot a permis de confirmer le diagnostic de la MJC (cas certains). Lors de l'autopsie, le LCR prélevé est immédiatement réparti dans les tubes décrits précédemment, sans centrifugation au préalable (à l'exception des échantillons présentant des débris cellulaires en quantité importante), et les aliquotes sont congelés à -80°C.
D'autre part, les échantillons négatifs pour la MCJ. Ils sont de 2 types :

LCR post-mortem récupérés lors de l'autopsie et dont la recherche de la $prp^{sc}$ dans le tissu cérébral par western blot a permis d'éliminer le diagnostic de la MJC (témoins négatifs pour la MJC)
LCR de patients non atteints de MJJ et issus de dérivations ventriculaires externes (DVE). Les conditions de stockage de ces prélèvements sont identiques à celles décrites précédemment.

6.2 Approche ELISA

a) principe analytique

Les échantillons sont simplement incubés à température élevée pendant un temps donné puis, après refroidissement, ils sont dilués au 1/2 ou au 1/5 dans un tampon compatible avec une analyse immunométrique (ELISA) (tampon Tris-Maléate pH 8). Lorsque la digestion par la protéinase K intervient avant le dépôt sur plaque C6S, les échantillons sont digérés par la protéinase K pendant 15 minutes puis sont déposés ainsi sur plaque C6S sans inhibition au préalable.
Le terme ELISA est employé bien que la capture ne soit pas immunologique car il s'agit d'une réaction de type ligand/affinant avec révélation immunologique.
Les techniques ELISA sont des techniques immuno-enzymologiques quantitatives, permettant de doser l'antigène recherché par la transformation d'un substrat en un produit soluble mesurable et proportionnel à la quantité d'antigène présent dans l'échantillon. La technique utilisée ici comporte les mêmes étapes qu'un ELISA sandwich non compétitif classique à l'exception de la sensibilisation des cupules. En effet, l'anticorps anti-prion de capture est remplacé par les Calix-6-Arènes sulfonés, greffés sur les groupements NHS des microplaques par leur fonction amine.
Après immobilisation des C6S au fond des cupules d'une microplaque et saturation des sites non spécifiques de fixation de l'antigène, l'échantillon est déposé et la plaque est incubée pour permettre la liaison de l'antigène avec le C6S. Puis, après plusieurs lavages, l'anticorps de révélation, spécifique de la protéine prion et marqué, est ajouté. Les complexes formés sont visualisés par méthode colorimétrique, après ajout du substrat de l'enzyme, qui se transforme en produit coloré, dont l'absorbance est déterminée à l'aide d'un lecteur de microplaque (spectrophotomètre automatisé).

b) Mode opératoire

Un volume de LCR est incubé au bain-marie sec à 75°C pendant 15 minutes. Après refroidissement, l'échantillon est dilué 5 fois dans du tampon Tris-Maléate 0,2 M, pH 8 (ajout de 4 volumes de tampon) et est, soit déposé directement sur une microplaque sensibilisée au préalable par les C6S, soit digéré par la protéinase K pendant 15 minutes à 37°C, puis déposé sur microplaque.
Les étapes successives de l'analyse immunométrique sont énumérées ci-dessous dans l'ordre chronologique :

1 - Le greffage des Calix-6-arènes sulfonés C6S sur les plaques amine activées (NHS) est réalisé suivant la méthode mentionnée ci-dessus durant 6 heures à température ambiante selon une gamme de concentration ou selon une concentration fixe, dilués dans une solution de PBS 50 mM.
2 - Les plaques sont ensuite lavées 3 fois en PBST 0,05% puis saturées en PBST 0,05%-Lait 5% pendant 1 heure à 37°C.
3 - Après lavage, 100 μL de LCR préparé selon le principe analytique décrit ci-dessus sont déposés par puits ; les plaques sont incubées pendant 1h30 à 37°C.
4 - Après lavage, 100 μL de l'anticorps de révélation sont déposés par puits (AC23-HRP, HPR pour peroxydase de raifort, à 0,5 μg/mL ou AC23-Biotine à 0,5 μg/mL). Les plaques sont incubées pendant 1 heure à température ambiante.
5 - Après lavage, les complexes formés sont révélés :

5.1 - Si l'anticorps de révélation est couplé à la biotine, 100 μL d'une solution de SA-PAL diluée au 1/20000

en tampon PBST 0,05% sont déposés par puits. Les plaques sont incubées pendant 20 minutes à température ambiante. Après lavage, 100 μL de PNPP sont déposés par puits. Les plaques sont incubées pendant 10 à 30 minutes à 37°C. A l'issue de l'incubation, la réaction colorimétrique est stoppée par l'ajout dans chaque puits de 50 μL de NaOH à 0,4 N. La densité optique est ensuite mesurée au spectrophotomètre à 405 nm contre un filtre à 450 nm.
5.2 - Si l'anticorps de révélation est couplé à la peroxydase, 100 μL d'OPD sont déposés par puits. Les plaques sont incubées pendant 10 minutes à l'obscurité et à température ambiante.

A l'issue de l'incubation, la réaction colorimétrique est stoppée par l'ajout dans chaque puits de 50 μL d'$H_2SO_4$ à 1,8 N. La densité optique est ensuite mesurée au spectrophotomètre à 495 nm.

b) Résultats

1 - Vérification de l'association des Calix-6-Arènes sulfonés mono-amines (C6S) greffés sur microplaque avec la protéine prion du liquide céphalo-rachidien (LCR) :

Un liquide céphalo-rachidien de patient non atteint de la maladie de Creutzfeld-Jakob est chauffé pendant 15 minutes à 75°C et est ensuite dilué au 1/2 ou au 1/6 dans un tampon compatible avec une révélation immunologique en ELISA. Les échantillons ne sont pas digérés par la protéinase K et sont déposés ainsi sur une plaque greffée avec des C6S selon une gamme de concentration comprise entre 1,8 et 18 μg/mL. Après incubation, la révélation immunologique de l'antigène capturé sur les C6S est assurée par l'anticorps AC23 couplé à la peroxydase, utilisé à 0,5 μg/mL. Les résultats sont présentés sur la figure 10.
Ces résultats montrent que les C6S s'associent bien avec une forme de protéine prion dans le LCR puisqu'un signal est observé dès la première concentration de C6S immobilisés au fond des cupules.

2 - Vérification de l'association des C6S avec la protéine prion pathologique en phase hétérogène sans utilisation de protéinase K:
Un LCR de patient non atteint de MJJ (LCR-) et un LCR de patient décédé de MJC (MJC+) non dilués ou dilués au 1/2 subissent différents types de traitement par la chaleur. Ils sont ensuite déposés sur plaque greffée avec les C6S à 7,2 μg/mL. Après lavage, la révélation immunologique est assurée par l'anticorps anti-prion AC23 couplé à la biotine.
Les résultats sont représentés graphiquement en figure 11.
Ces résultats montrent une adsorption de la protéine prion sur les C6S greffés sur la plaque en absence de traitement par la protéinase K plus efficace pour l'échantillon positif que pour l'échantillon négatif. Les différents traitements par la chaleur influencent légèrement l'efficacité d'adsorption de la protéine prion sur les C6S en absence de dilution et davantage lorsque l'échantillon est dilué. Ainsi, les conditions expérimentales favorisant l'adsorption de la protéine prion sur les C6S MN en phase hétérogène semblent être une dilution des échantillons et un traitement pendant 5 minutes à 95°C.
3 - Vérification de l'association des C6S avec la protéine prion pathologique en phase hétérogène avec utilisation de protéinase K:

On a utilisé une étape de digestion par la protéinase K pendant 15 minutes à 37°C en traitement pré-analytique, avant capture sur plaque C6S du mélange digestionnel pendant 1 heure à 37°C. L'élimination de la protéinase K résiduelle est assurée par des lavages successifs en tampon PBS-Tween 0,05%.
Les résultats sont indiqués sur la figure 12.
Ces résultats montrent une différence de densité optique en faveur de l'échantillon positif pour la MJC après digestion par la protéinase K. Ce différentiel est visible dès la première concentration de protéinase K testée (0,5 μg/mL).

d) Conclusion

La détection de la protéine prion dans le LCR est rendue possible selon le procédé de l'invention. En effet, en absence de digestion par la protéinase K, la détection de la PrP totale (cellulaire et pathologique) est amplifiée en présence des Calix-6-Arènes sulfonés et, de façon inattendue, la $PrP^{sc}$ est préférentiellement détectée. L'utilisation de ces C6S en capture, après digestion des échantillons par la protéinase K, permet de détecter la $prp^{sc}$ du LCR en fournissant un signal significativement différent entre les LCR issus de patients non atteints de MJC et les LCR issus

de patients atteints de MJC.

## Revendications

1. Procédé de détection de la PrP, et en particulier de la $PrP^{sc}$, **caractérisé en ce que** l'on ajoute dans un échantillon biologique susceptible de contenir de la $PrP^{sc}$, un ligand adjuvant macrocyclique (AML) libre ou lié à un support, puis on fait réagir la suspension résultante avec un anticorps anti-$PrP^{sc}$, et on détecte la présence de PrP.

2. Procédé de détection de la PrP, et en particulier de la $PrP^{sc}$ selon la revendication 1, **caractérisé en ce qu'**il comprend l'étape supplémentaire de traitement de l'échantillon avec de la protéinase K avant ou après contact avec l'AML.

3. Procédé de détection de la PrP, et en particulier de la $PrP^{sc}$ selon l'une des revendications 1 ou 2, **caractérisé en ce que** ledit échantillon biologique provient d'un animal, y compris l'homme.

4. Procédé de détection de la PrP, et en particulier de la $Prp^{sc}$ selon la revendication 3, **caractérisé en ce que** ledit échantillon est un tissu ou un fluide biologique, notamment le liquide céphalo-rachidien ou le sérum.

5. Procédé de détection de la PrP, et en particulier de la $prp^{sc}$ selon l'une quelconque des revendications 1 à 4, **caractérisé en ce que** ledit support solide est de préférence fonctionnalisé par un groupe NHS ou un groupe $NH_2$ et est de préférence une bille magnétique ou une microplaque.

6. Procédé de détection de la PrP, et en particulier de la $PrP^{sc}$ selon l'une quelconque des revendications 1 à 5, **caractérisé en ce que** ledit ligand adjuvant macrocyclique se lie à des sites contigus ou proches dans l'espace des sites de liaison de la $PrP^{sc}$ avec un anticorps.

7. Procédé de détection de la PrP, et en particulier de la $PrP^{sc}$ selon l'une quelconque des revendications 1 à 6, **caractérisé en ce que** le ligand adjuvant macrocyclique est choisi dans la famille des métacyclophanes et en particulier parmi les calix-arènes.

8. Procédé de détection de la PrP, et en particulier de la $PrP^{sc}$ selon la revendication 7, **caractérisé en ce que** les calix-arènes préférés sont les para-sulfoanto-calix-arènes fonctionnalisés sur la face phénolique, de préférence le p-sulphonato-calix-[4]-arène, le p-sulphonato-calix-[6]-arène, le p-sulphonato-calix-[8]-arène, ou l'un de leurs dérivés.

9. Procédé de détection de la PrP, et en particulier de la $prp^{sc}$ selon l'une quelconque des revendications 7 ou 8, **caractérisé en ce que** le ligand adjuvant macrocyclique répond à la formule générale (I) ci-dessous

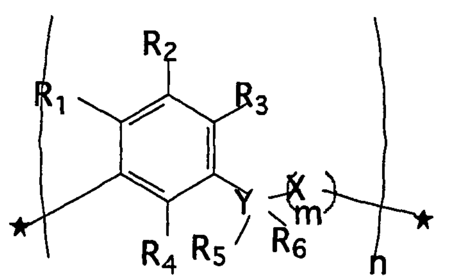

(I)

dans laquelle

$R_1$ représente un atome d'hydrogène, un groupe hydroxyle, un groupe OR ou un groupe OCOR, R étant tel que défini ci-dessous,
$R_2$ représente un atome d'hydrogène, un groupe R, COR, Pol, $CH_2Pol$, dans lesquels Pol représente un groupe phosphate, sulfate, amine, ammonium, acide carboxylique et R est tel que défini ci-dessous,
$R_3$ représente un atome d'hydrogène, un groupe hydroxyle, un groupe OR ou un groupe OCOR dans lesquels

R est tel que défini ci-dessous,
$R_4$ représente un atome d'hydrogène, un groupe hydroxyle, un groupe OR, un groupe $OCH_2R$ ou un groupe OCOR, dans lesquels R est tel que défini ci-dessous,
Y est un atome de carbone, d'azote, ou un atome de soufre,
$R_5$ et $R_6$, chacun indépendamment, sont absents ou représentent un atome d'hydrogène, un groupement $CH_2$ ou R tel que défini ci-dessous, ou bien
$R_5$ et $R_6$ représentent ensemble un atome d'oxygène ou de soufre,
X représente un groupement $CH_2$, ou un atome d'oxygène ou de soufre,
m représente un entier égal à 0 ou 1,
R représente un atome d'hydrogène ou une chaîne hydrocarbonée, saturée ou non, ramifiée ou non, cyclique ou non cyclique, substituée ou non par un groupement halogène, et portant des fonctions polaires ou non polaires,
n est un entier compris entre 3 et 15,

les substituants $R_1$ à $R_5$, R, X, Y et l'entier m peuvent être de nature différente suivant les motifs.

**10.** Procédé de détection de la PrP, et en particulier de la $PrP^{sc}$ selon la revendication 9, **caractérisé en ce que** ledit ligand adjuvant macrocyclique répond à la formule générale (Ia) ci-dessous

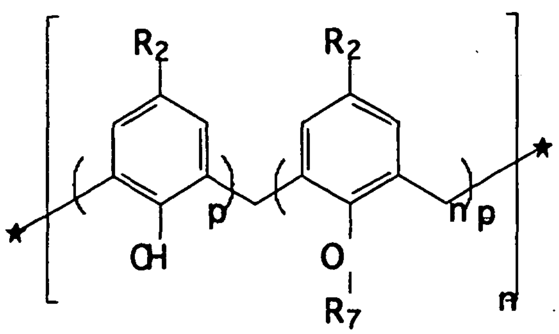
(Ia)

dans laquelle

chaque groupement $R_2$, pris indépendamment, est un groupement sulfate ou un groupement phosphate,
$R_7$ est un groupement $(CH_2)_r$-Z dans lequel Z est un groupement COOEt, COOH, CN ou $NH_2$,
r est un entier compris entre 1 et 20,
p est un entier compris entre 1 et 10,
n est un entier compris entre 2 et 10.

**11.** Procédé selon la revendication 9, **caractérisé en ce que** ledit ligand répond à la formule (Ib) ci-dessous :

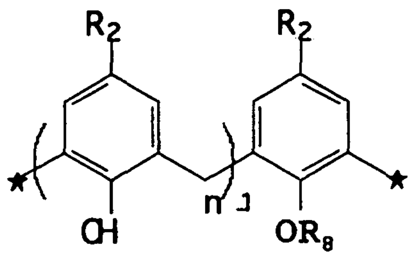
(Ib)

dans laquelle

n est un entier compris entre 4 et 8,
chaque groupement $R_2$, pris indépendamment, est un groupement sulfate ou un groupement phosphate,
$R_8$ représente un groupement $(CH_2)_t$-$(CO)_s$-$(NH_2)$ ou un groupement $(CH_2)_t$-COOH où t est un entier compris

entre 0 et 6 et s est un entier compris entre 0 et 6.

**12.** Procédé selon la revendication 11, **caractérisé en ce que** ledit ligand est un calix-arène de formule (Ib) dans lequel les deux groupements $R_2$ sont chacun un groupement sulfate, n est 4, 6 ou 8, et $R_8$ est un atome d'hydrogène, un groupement $CH_2COOH$, un groupement $CH_2CONH_2$ ou un groupement $CH_2CH_2NH_2$.

**13.** Procédé selon la revendication 11, **caractérisé en ce que** ledit ligand est tel que les deux groupements $R_2$ sont chacun un groupement sulfate, n est un entier égal à 6, et $R_8$ est un groupement $(CH_2)_2$-$NH_2$.

**14.** Procédé de détection de la PrP, et en particulier de la $prp^{sc}$ selon l'une quelconque des revendications 7 à 12, **caractérisé en ce que** ledit calixarène est lié à un support fonctionnalisé notamment au moyen d'une fonction NHS ou $NH_2$.

**15.** Ligand adjuvant macrocyclique greffé sur un support fonctionnalisé, dans lequel le ligand répond à la formule (Ib)

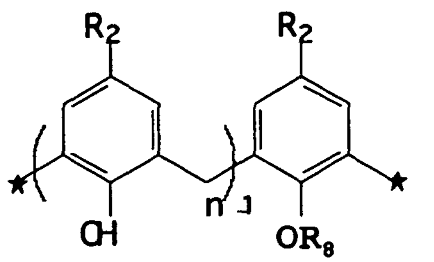

(Ib)

dans laquelle dans laquelle n est un entier compris entre 4 et 8,
chaque groupement $R_2$, pris indépendamment, est un groupement sulfate ou un groupement phosphate,
$R_8$ représente un groupement $(CH_2)_t$ - $(CO)_s$ - $(NH_2)$ ou un groupement $(CH_2)_t$ - COOH où t est un entier compris entre 0 et 6 et s est un entier compris entre 0 et 6,

et le support est du type support solide de préférence fonctionnalisé par un groupe NHS ou un groupe $NH_2$ et est de préférence une bille magnétique ou une microplaque.

**16.** Utilisation d'un ligand adjuvant macrocyclique pour la détection de la PrP, de la PrP recombinante, physiologique ou pathologique, bovine ou humaine ou d'autres espèces, $PrP^{sc}$ dans un échantillon biologique, notamment un fluide biologique, et en particulier d'un ligand de formule (I), (Ia) ou (Ib) tel que défini dans les revendications 9, 10 et 15 libre ou lié à un support.

**17.** Utilisation d'un kit comprenant un ligand adjuvant macrocyclique, et en particulier un ligand de formule (I), (Ia) ou (Ib) tel que défini dans les revendications 9, 10 et 15, libre ou lié à un support, pour le diagnostic des maladies dont le $prp^{sc}$ est responsable, du type ESB, la tremblante de petits ruminants, Creutzfeld-Jakob.

**18.** Utilisation d'un kit comprenant un ligand adjuvant macrocyclique, et en particulier un ligand de formule (I), (Ia) ou (Ib) tel que défini dans les revendications 9, 10 et 15, libre ou lié à un support, pour le dosage immunologique de la $PrP^{sc}$.

**Claims**

**1.** A method of detecting prion protein (PrP) and in particular the ($PrP^{Sc}$) **characterized in that** is added to a biological sample that might contain $PrP^{Sc}$, an ancillary macrocyclic ligand (AML) that is free or that is bonded to a support then in causing the resulting suspension to react with an anti-$prp^{Sc}$ antibody, and in detecting any presence of PrP.

**2.** A method of detecting PrP and in particular $PrP^{Sc}$ according to claim 1, **characterized in that** it further comprises

an additional step of treating the sample with the proteinase K before or after contact with the AML.

3. A method of detecting PrP and in particular $PrP^{Sc}$ according to claim 1 or claim 2, **characterized in that** said biological sample comes from an animal, including a human.

4. A method of detecting PrP and in particular $PrP^{Sc}$ according to claim 3, **characterized in that** said sample is a biological tissue or a biological fluid, in particular cerebrospinal fluid or serum.

5. A method of detecting PrP and in particular $PrP^{Sc}$ according to any one of claims 1 to 4, **characterized in that** said solid support is preferably functionalized by an NHS group or by an $NH_2$ group, and is preferably a magnetic ball or a micro-plate.

6. A method of detecting PrP and in particular $PrP^{Sc}$ according to any one of claims 1 to 5, **characterized in that** said ancillary macrocyclic ligand is bonded to sites that are spatially contiguous to or near to sites where $PrP^{Sc}$ bonds with an antibody.

7. A method of detecting PrP and in particular $PrP^{Sc}$ according to any one of claims 1 to 6, **characterized in that** the ancillary macrocyclic ligand is chosen from the family of metacyclophanes and in particular from calixarenes.

8. A method of detecting PrP and in particular $prp^{Sc}$ according to claim 7, **characterized in that** the preferred calixarenes are para-sulfonato-calixarenes functionalized on the phenolic face, and preferably p-sulfonato-calix-[4]-arene, p-sulfonato-calix-[6]-arene, p-sulfonato-calix-[8]-arene, or any one of the derivatives thereof.

9. A method of detecting PrP and in particular $PrP^{Sc}$ according to claim 7 or claim 8, **characterized in that** the ancillary macrocyclic ligand satisfies the following general formula (I) :

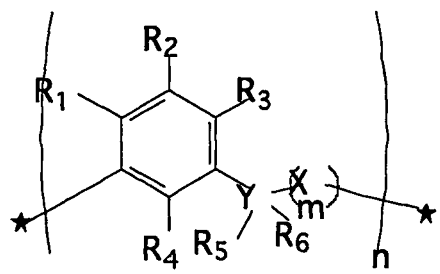

(I)

where:

$R_1$ represents a hydrogen atom, a hydroxyl group, an OR group or an OCOR group, where R is as defined below;
$R_2$ represents a hydrogen atom, or an R, COR, Pol, or $CH_2Pol$ group, where Pol represents a phosphate, sulfate, amine, ammonium, or carboxylic acid group, and R is as defined below;
$R_3$ represents a hydrogen atom, a hydroxyl group, an OR group, or an OCOR group, where R is as defined below;
$R_4$ represents a hydrogen atom, a hydroxyl group, an OR group, an $OCH_2R$ group, or an OCOR group, where R is as defined below;
Y is a carbon atom, a nitrogen atom, or a sulfur atom;
$R_5$ and $R_6$, each independently, are absent or represent a hydrogen atom, a $CH_2$ group or an R group as defined below, or else:

$R_5$ and $R_6$ together represent an oxygen atom or a sulfur atom;

X represents a $CH_2$ group, or an oxygen atom or a sulfur atom;
m represents an integer equal to 0 or 1;
R represents a hydrogen atom or a hydrocarbonated chain, saturated or otherwise, branched or otherwise, cyclic or non-cyclic, substituted or not substituted with a halogen group, and carrying polar or non-polar functions;
n is an integer lying in the range 3 to 15; and

the substituents $R_1$ to $R_5$, R, X, Y and the integer m may be of different types depending on the units.

**10.** A method of detecting PrP and in particular prp$^{sc}$ according to claim 9, **characterized in that** said ancillary macrocyclic ligand satisfies the following general formula (Ia) :

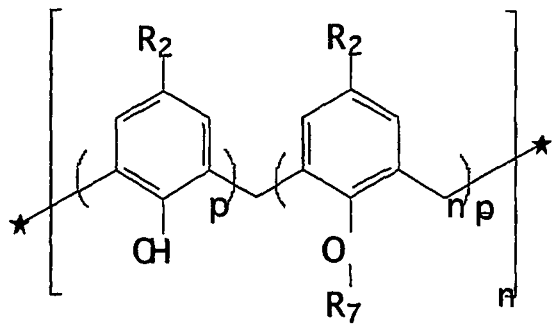

(Ia)

where:

each $R_2$ group, taken independently, is a sulfate group or a phosphate group;
$R_7$ is a group $(CH_2)_r$-Z in which Z is a COOEt, COOH, CN or $NH_2$ group;
r is an integer lying in the range 1 to 20;
p is an integer lying in the range 1 to 10; and
n is an integer lying in the range 2 to 10.

**11.** A method according to claim 9, **characterized in that** said ligand satisfies the following formula (Ib):

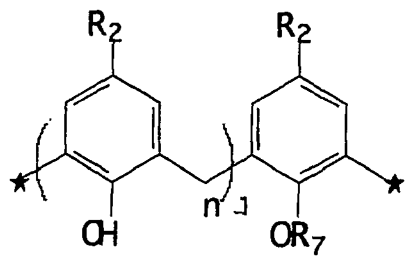

(Ib)

where:

n is an integer lying in the range 4 to 8;
each group $R_2$, taken independently, is a sulfate group or a phosphate group;
$R_8$ represents a $(CH_2)_t$- (CO) $_s$- ($NH_2$) group or a $(CH_2)_t$-COOH, group, where t is an integer lying in the range 0 to 6, and s is an integer lying in the range 0 to 6.

**12.** A method according to claim 11, **characterized in that** said ligand is a calixarene of formula (Ib) in which each of the two $R_2$ groups is a sulfate group, n is 4, 6 or 8, and $R_8$ is a hydrogen atom, a $CH_2COOH$ group, a $CH_2CONH_2$ group, or a $CH_2CH_2NH_2$ group.

**13.** A method according to claim 11, **characterized in that** said ligand is such that each of the two $R_2$ groups is a sulfate group, n is an integer equal to 6, and $R_8$ is a $(CH_2)_2$-$NH_2$ group.

**14.** A method of detecting PrP, and in particular prp$^{sc}$ according to any one of claims 7 to 12, **characterized in that** said calixarene is bonded to a support that is functionalized in particular by means of an NHS or $NH_2$ function.

**15.** An ancillary macrocyclic ligand grafted onto a functionalized support, wherein the ligand satisfies the formula (Ib) below:

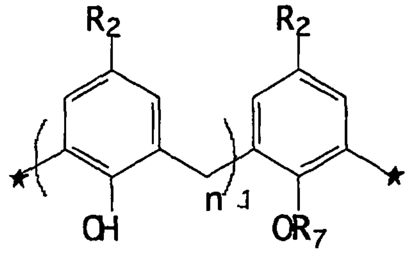

(Ib)

where n is an integer lying in the range 4 to 8;
each $R_2$ group, taken independently, is a sulfate group or a phosphate group;
$R_8$ represents a $(CH_2)_t$-$(CO)_s$-$(NH_2)$ group or a $(CH_2)_t$-COOH group where t is an integer lying in the range 0 to 6, and s is an integer lying in the range 0 to 6; and

the support is of the solid support type that is preferably functionalized by an NHS group or by an $NH_2$ group, and is preferably a magnetic ball or a micro-plate.

**16.** The use of an ancillary macrocyclic ligand for detecting PrP, recombinant, physiological, or pathological bovine, human, or other species PrP, or $PrP^{Sc}$, in a biological sample, in particular a biological fluid, and in particular the use of a ligand of formula (I), (Ia) or (Ib) as defined in claims 9, 10, and 15 and that is free or that is bonded to a support.

**17.** The use of a kit including an ancillary macrocyclic ligand, and in particular a ligand of formula (I), (Ia) or (Ib) as defined in claims 9, 10, and 15 and that is free or that is bonded to a support, for diagnosing diseases for which $PrP^{Sc}$ is responsible, of the following types: Bovine Spongiform Encephalopathy (BSE), scrapie in small ruminants, and Creutzfeldt-Jakob Disease (CJD).

**18.** The use of a kit including an ancillary macrocyclic ligand, and in particular a ligand of formula (I), (Ia) or (Ib) as defined in claims 9, 10, and 15 and that is free or that is bonded to a support, for immunological assay of $Prp^{Sc}$.

**Patentansprüche**

**1.** Verfahren zum Nachweis von PrP, und insbesondere $PrP^{Sc}$, **dadurch gekennzeichnet, dass** man zu einer biologischen Probe, die $PrP^{Sc}$ enthalten könnte, als Adjuvans einen makrozyklischen Liganden (AML) hinzufügt, der an einen Träger gebunden sein kann, man die daraus resultierende Suspension mit einem anti-$PrP^{Sc}$ Antikörper reagieren lässt, und das Vorhandensein von PrP nachweist;

**2.** Verfahren zum Nachweis von PrP und insbesondere von $PrP^{Sc}$ gemäß Patentanspruch 1, **dadurch gekennzeichnet, dass** es eine zusätzliche Phase umfasst, die aus der Behandlung der Probe mit Proteinase K vor oder nach dem Kontakt mit dem AML besteht.

**3.** Verfahren zum Nachweis von PrP und insbesondere von $prp^{sc}$ gemäß Patentanspruch 1 oder 2, **dadurch gekennzeichnet, dass** besagte biologische Probe von einem Tier, aber auch von einem Menschen stammen kann.

**4.** Verfahren zum Nachweis von PrP und insbesondere von $prp^{sc}$ gemäß Patentanspruch 3, **dadurch gekennzeichnet, dass** besagte Probe ein Gewebe oder eine Körperflüssigkeit ist, insbesondere eine zerebrospinale Flüssigkeit oder ein Serum.

**5.** Verfahren zum Nachweis von PrP und insbesondere von $PrP^{Sc}$ gemäß einem der Patentansprüche 1 bis 4, **dadurch gekennzeichnet, dass** besagter fester Träger vorzugsweise durch eine Gruppe NHS oder eine Gruppe $NH_2$ funk-

tionalisiert wird und vorzugsweise eine Magnetkugel oder eine Mikroplatte ist.

6. Verfahren zum Nachweis von PrP und insbesondere von $PrP^{Sc}$ gemäß einem der Patentansprüche 1 bis 5, **dadurch gekennzeichnet, dass** sich der als Adjuvans verwendete besagte makrolytische Ligand an aneinandergrenzenden oder räumlich nahe beieinander liegenden Stellen im Bereich der Bindungsstellen von $PrP^{Sc}$ an einen Antikörper bindet.

7. Verfahren zum Nachweis von PrP und insbesondere von $PrP^{Sc}$ gemäß einem der Patentansprüche 1 bis 6, **dadurch gekennzeichnet, dass** der als Adjuvans verwendete makrolytische Ligand aus der Familie der Metazyklophane und insbesondere aus der Familie der Calixarene ausgewählt wird.

8. Verfahren zum Nachweis von PrP und insbesondere von $PrP^{Sc}$ gemäß Patentanspruch 7, **dadurch gekennzeichnet, dass** die bevorzugten Calixarene Para-Sulfonat-Calixarene sind, die auf der Phenolseite, vorzugsweise von Calix[4]aren-p-Sulfonat, Calix[6]aren-p-sulfonat, Calix[8]aren-p-sulfonat oder einem ihrer Derivate funktionalisiert wird.

9. Verfahren zum Nachweis von PrP und insbesondere von $prp^{sc}$ gemäß einem der Patentansprüche 7 oder 8, **dadurch gekennzeichnet, dass** der als Adjuvans verwendete makrozyklische Ligand nachstehende allgemeine Formel hat (I) :

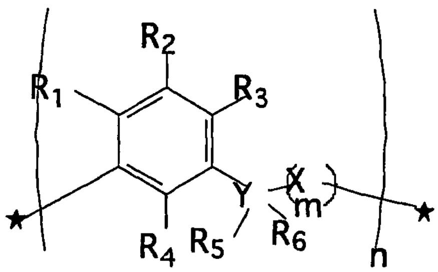

(I)

in der

$R_1$ ein Wasserstoffatom, eine Hydroxylgruppe, eine Gruppe OR oder eine Gruppe OCOR ist, und R der Definition unten entspricht,
$R_2$ ein Wasserstoffatom, eine Gruppe R, COR, Pol, $CH_2Pol$ ist, in der Pol eine Phosphat-, Sulfat-, Amin-, Ammonium-, Carbonsäure-Gruppe ist und R der Definition unten entspricht,
$R_3$ ein Wasserstoffatom, eine Hydroxylgruppe, eine Gruppe OR oder OCOR ist, und R der Definition unten entspricht,
$R_4$ ein Wasserstoffatom, eine Hydroxylgruppe, eine Gruppe OR, $OCH_2R$ oder OCOR ist, in der R der Definition unten entspricht,
Y ein Kohlenstoff-, Stickstoff- oder Schwefelatom ist.
$R_5$ und $R_6$ können jeweils einzeln betrachtet fehlen, oder ein Wasserstoffatom, eine CH - Gruppe oder R gemäß der Definition unten sein, oder
$R_5$ und $R_6$ bilden zusammen ein Sauerstoff- oder Schwefelatom,
m ist eine ganze Zahl gleich 0 oder 1,
R ist ein Wasserstoffatom oder eine gesättigte oder ungesättigte, verzweigte oder unverzweigte, zyklische oder nicht zyklische Kohlenwasserstoffkette, die durch eine Halogengruppe ersetzt werden und Polfunktionen haben kann,
n ist eine ganze Zahl zwischen 3 und 15,

die Substituenten $R_1$ bis $R_5$, R, X, Y und die ganze Zahl m können je nach Muster unterschiedlicher Art sein.

10. Verfahren zum Nachweis von PrP und insbesondere von $prp^{Sc}$ gemäß Patentanspruch 9, **dadurch gekennzeichnet, dass** besagter als Adjuvans verwendeter makrozyklischer Ligand nachstehende allgemeine Formel (Ia) hat:

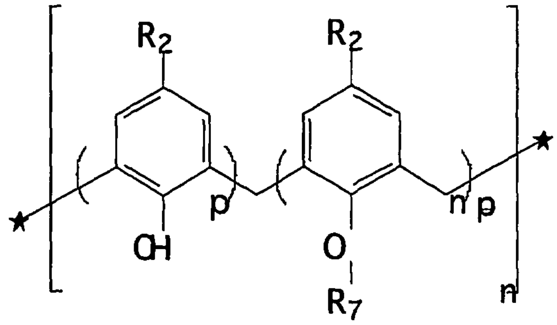

(Ia)

in der jede $R_2$-Gruppe einzeln betrachtet eine Sulfat- oder eine Phosphatgruppe ist,
$R_7$ eine Gruppe $(CH_2)$ r-Z ist, in der Z eine Gruppe COOEt, COOH, CN oder $NH_2$ ist,
r eine ganze Zahl zwischen 1 und 20 ist,
p eine ganze Zahl zwischen 1 und 10 ist,
n eine ganze Zahl zwischen 2 und 10 ist.

**11.** Verfahren gemäß Patentanspruch 9, **dadurch gekennzeichnet, dass** besagter Ligand nachstehende Formel (Ib) hat:

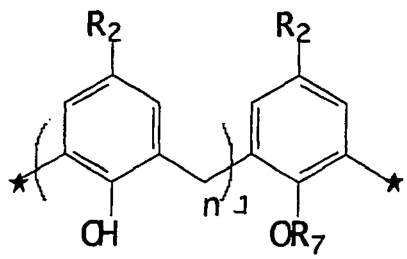

(Ib)

in der

n eine ganze Zahl zwischen 4 und 8 ist,
jede $R_2$-Gruppe einzeln betrachtet eine Sulfat- oder Phosphatgruppe ist,
$R_8$ eine Gruppe $(CH_2)$ t- (CO) 3- $(NH_2)$ oder eine Gruppe $(CH_2)$ t-COOH ist, in der t eine ganze Zahl zwischen 0 und 6 und s eine ganze Zahl zwischen 0 und 6 ist.

**12.** Verfahren nach Patentanspruch 11, **dadurch gekennzeichnet, dass** besagter Ligand ein Calixaren mit der Formel (Ib) ist, in dem die beiden $R_2$-Gruppen jeweils eine Sulfatgruppe sind, wobei n gleich 4, 6 oder 8 ist und $R_8$ ein Wasserstoffatom, eine Gruppe $CH_2COOH$, eine Gruppe $CH_2CONH_2$ oder eine Gruppe $CH_2CH_2NH_2$ ist.

**13.** Verfahren nach Patentanspruch 11, **dadurch gekennzeichnet, dass** besagter Ligand dergestalt ist, dass die beiden $R_2$-Gruppen jeweils eine Sulfatgruppe sind, wobei n eine ganze Zahl gleich 6, und $R_9$ eine Gruppe $(CH_2)_2$-$NH_2$ ist.

**14.** Verfahren zum Nachweis von PrP und insbesondere von $PrP^{sc}$ gemäß einem der Patentansprüche 7 bis 12, **dadurch gekennzeichnet, dass** besagtes Calixaren an einen Träger gebunden ist, der insbesondere durch eine Funktion NHS oder $NH_2$ funktionalisiert ist.

**15.** Als Adjuvans verwendeter makrozyklischer Ligand, der auf einen funktionalisierten Träger gepfropft wird, in dem der Ligand folgende Formel (Ib) hat:

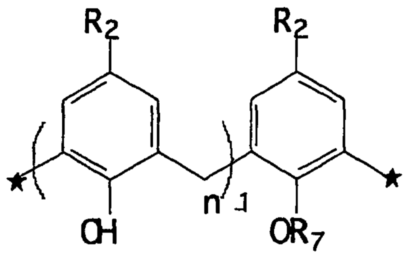

(Ib)

n eine ganze Zahl zwischen 4 und 8 ist,
jede $R_2$-Gruppe einzeln betrachtet eine Sulfat- oder eine Phosphatgruppe ist,
$R_8$ eine Gruppe $(CH_2)_t$-$(CO)_s$-$(NH_2)$ oder eine Gruppe $(CH_2)_t$-COOH ist, in der t eine ganze Zahl zwischen 0 und 6 und s eine ganze Zahl zwischen 0 und 6 ist,

und der Träger ein fester Träger ist, der vorzugsweise durch eine NHS- oder eine $NH_2$-Gruppe funktionalisiert ist und vorzugsweise ein Magnetkugel oder eine Mikroplatte ist.

**16.** Benutzung eines makrozyklischen Liganden als Adjuvans zum Nachweis von PrP, rekombinantem, physiologischem oder pathologischem PrP, vom Rind, vom Menschen oder anderen Spezies, und $PrP^{sc}$, in einer biologischen Probe, insbesondere einer Körperflüssigkeit, und insbesondere eines Liganden mit der Formel (I), (Ia) oder (Ib) gemäß der Definition in den Patentansprüchen 9, 10 und 15, der an einen Träger gebunden sein kann.

**17.** Benutzung eines Sets, das einen makrozyklischen Liganden als Adjuvans und insbesondere einen Liganden mit der Formel (I), (Ia) oder (Ib) gemäß der Definition in den Patentansprüchen 9, 10 und 15 umfasst, welcher an einen Träger gebunden sein kann, zur Diagnose von Krankheiten, für die $PrP^{Sc}$ verantwortlich ist, wie z.B. Krankheiten vom Typ BSE, die Traberkrankheit der Kleinwiederkäuer oder die Creutzfeld-Jakob-Krankheit.

**18.** Benutzung eines Sets mit einem makrozyklischen Liganden als Adjuvans und insbesondere einem Liganden mit der Formel (I), (Ia) oder (Ib) gemäß der Definition in den Patentansprüchen 9, 10 und 15, der an einen Träger gebunden sein kann, zur immunologischen Dosierung von $PrP^{Sc}$.

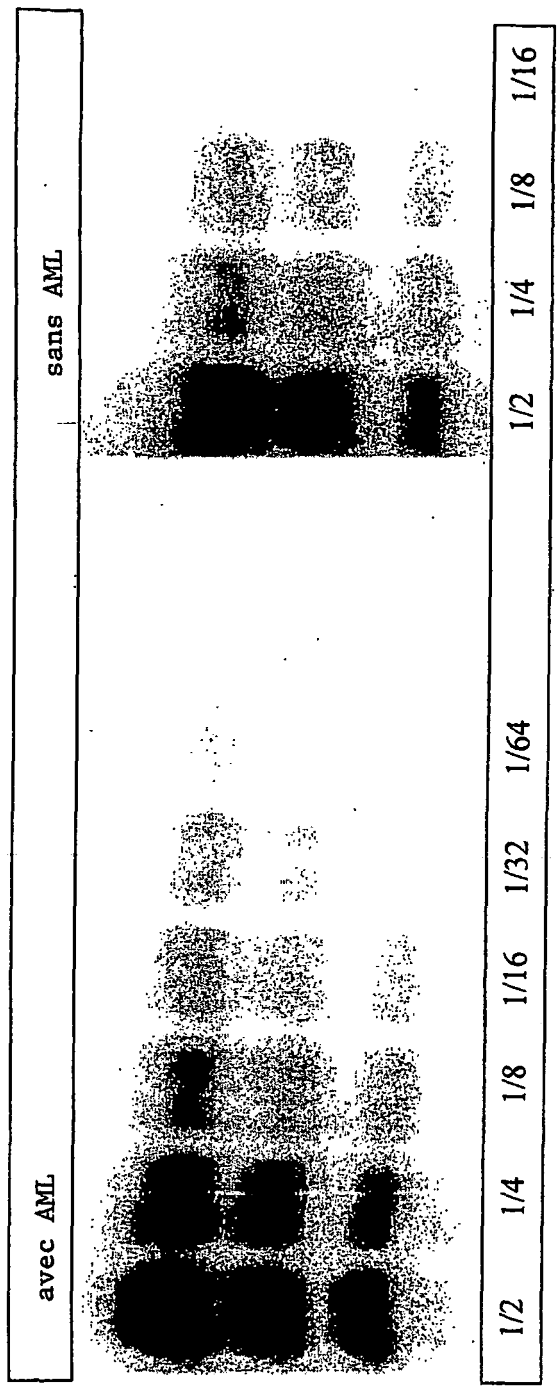


Figure 1

| | Avec AML | | | | | Sans AML | |
|---|---|---|---|---|---|---|---|
| Dilution | 1/8 | 1/16 | 1/32 | 1/64 | | 1 / 4 | 1/8 |
| Ligne | 1 | 2 | 3 | 4 | 5 | 6 | 7 |
| | 4257 | 1883 | 1331 | 538 | | 3074 | 1081 |
| | 3162 | 1190 | 761 | | | 2067 | 787 |
| | 1503 | 426 | 355 | | | 1184 | 501 |

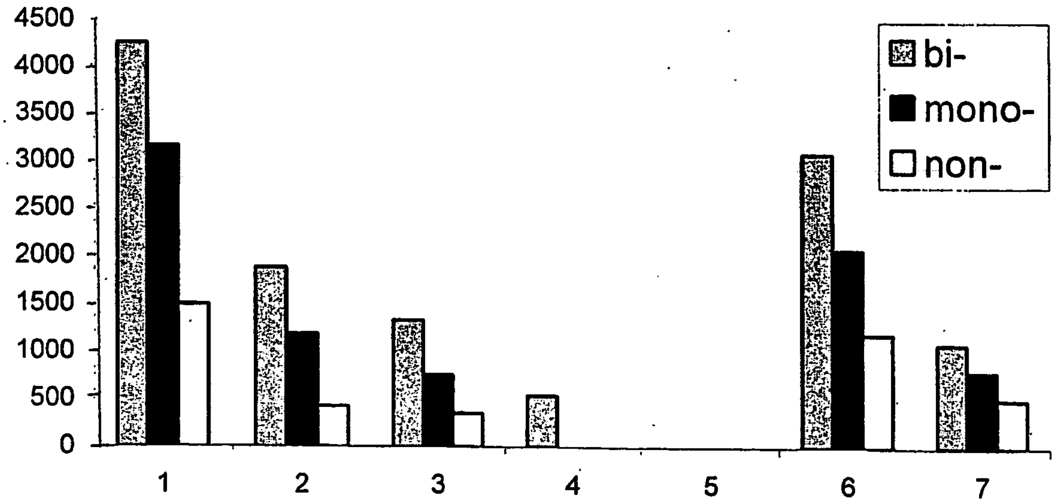


Figure 2

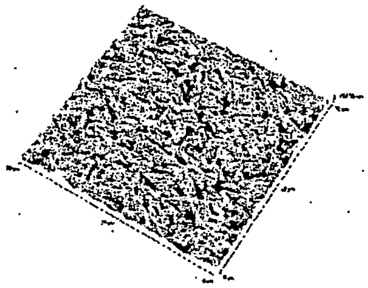

Figure 3 A

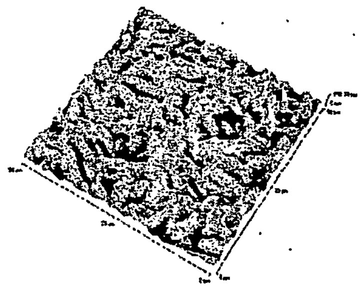

Figure 3 B

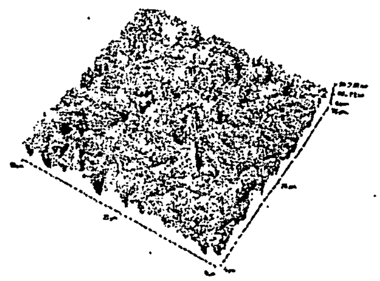

Figure 3 C

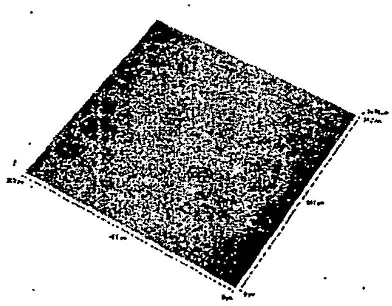

Figure 4 A

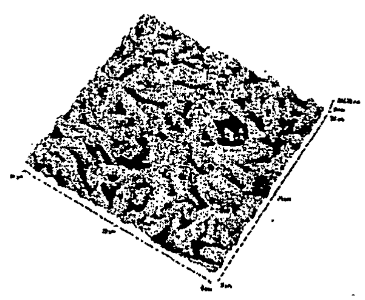

Figure 4 B

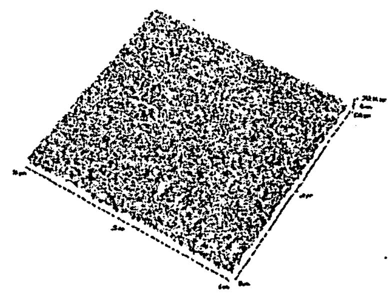

Figure 4 C

Figure 5

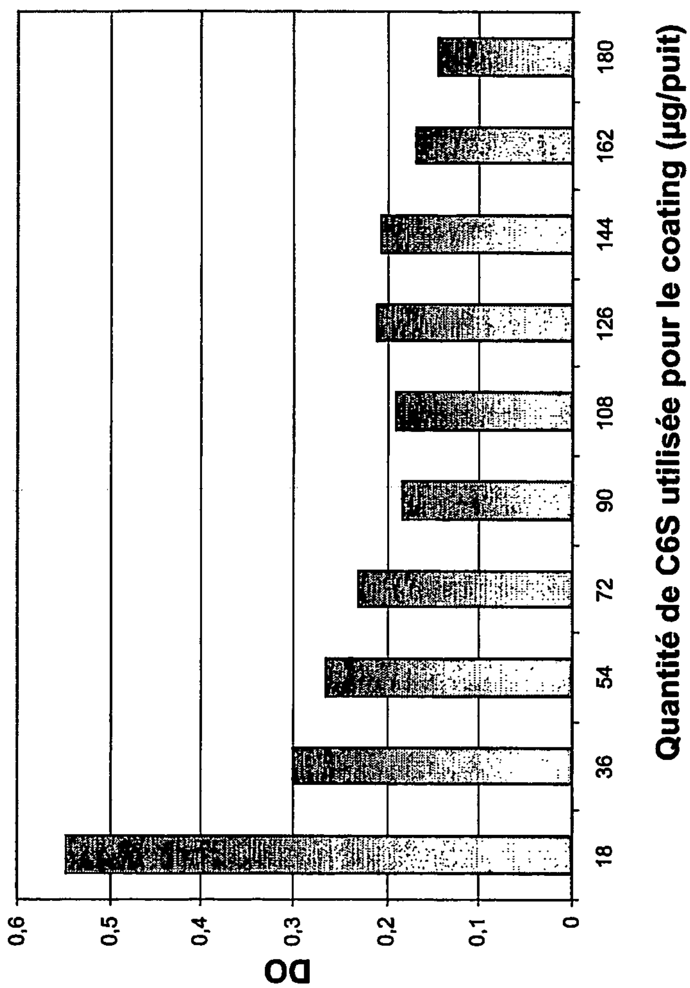
DO
0,6
0,5
0,4
0,3
0,2
0,1
0
18
36
54
72
90
108
126
144
162
180
Quantité de C6S utilisée pour le coating (µg/puit)

Figure 6

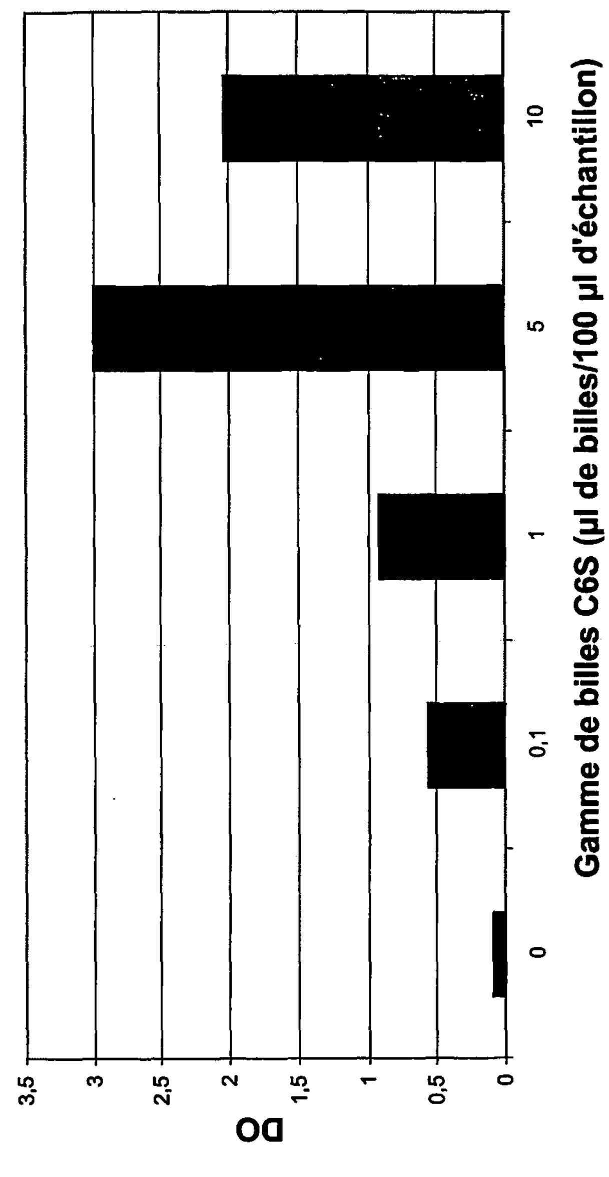
DO
3,5
3
2,5
2
1,5
1
0,5
0
0
0,1
1
5
10
Gamme de billes C6S (µl de billes/100 µl d'échantillon)

Figure 7

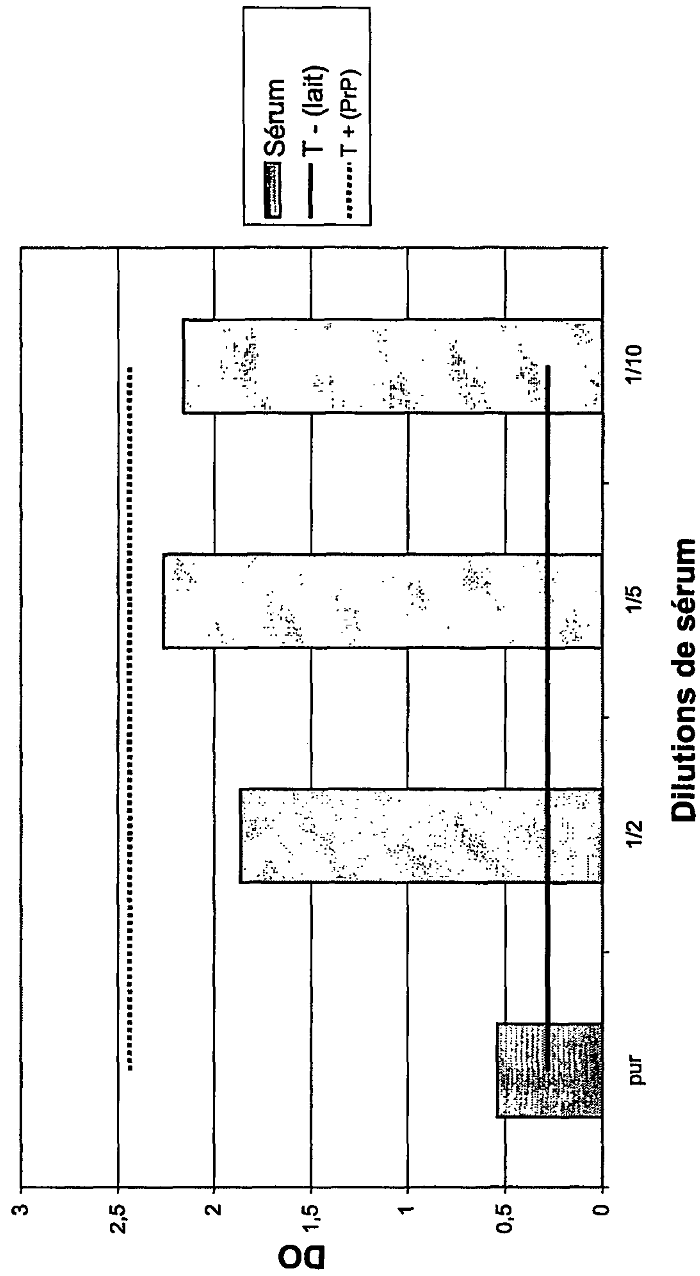
DO
3
2,5
2
1,5
1
0,5
0
pur
1/2
1/5
1/10
Dilutions de sérum
Sérum
T - (lait)
T + (PrP)

Figure 8

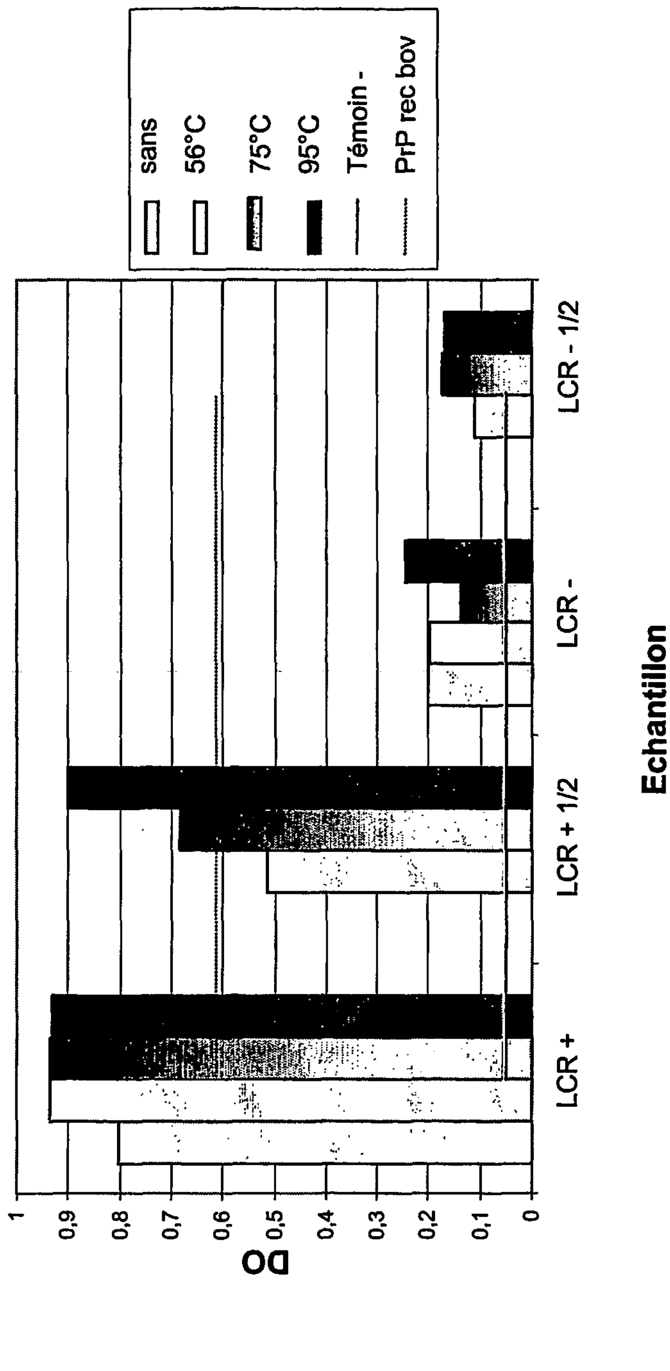
DO
1
0,9
0,8
0,7
0,6
0,5
0,4
0,3
0,2
0,1
0
LCR +
LCR + 1/2
LCR -
LCR - 1/2
Echantillon
sans
56°C
75°C
95°C
Témoin -
PrP rec bov

Figure 9

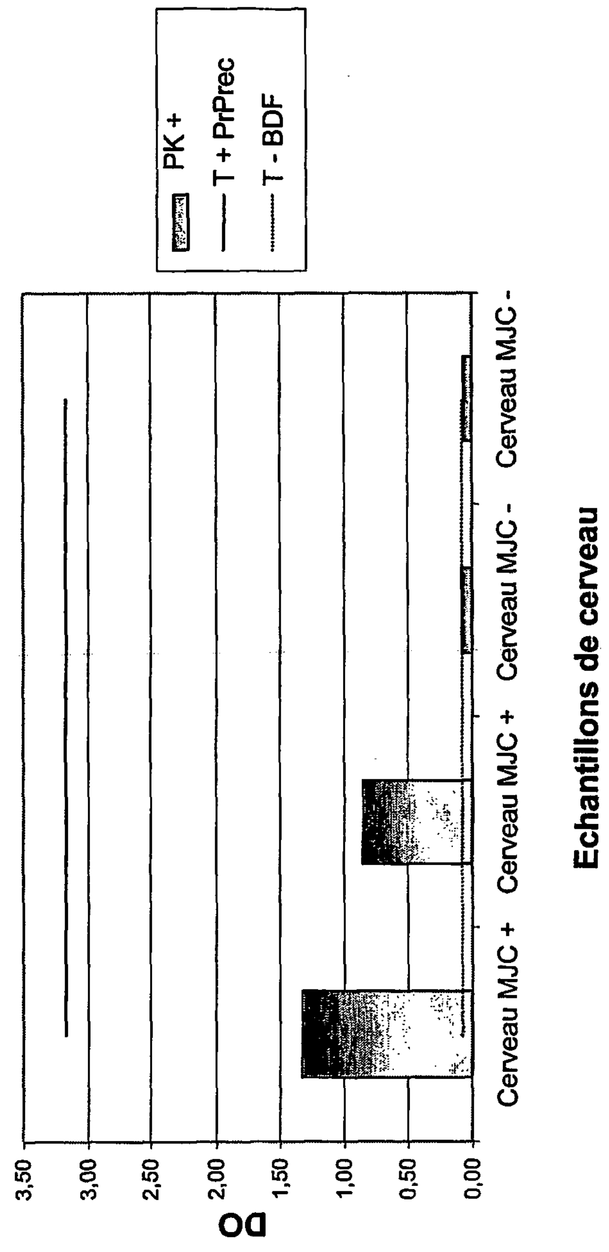
PK +
T + PrPrec
T - BDF
DO
3,50
3,00
2,50
2,00
1,50
1,00
0,50
0,00
Cerveau MJC +
Cerveau MJC +
Cerveau MJC -
Cerveau MJC -
Echantillons de cerveau

Figure 10

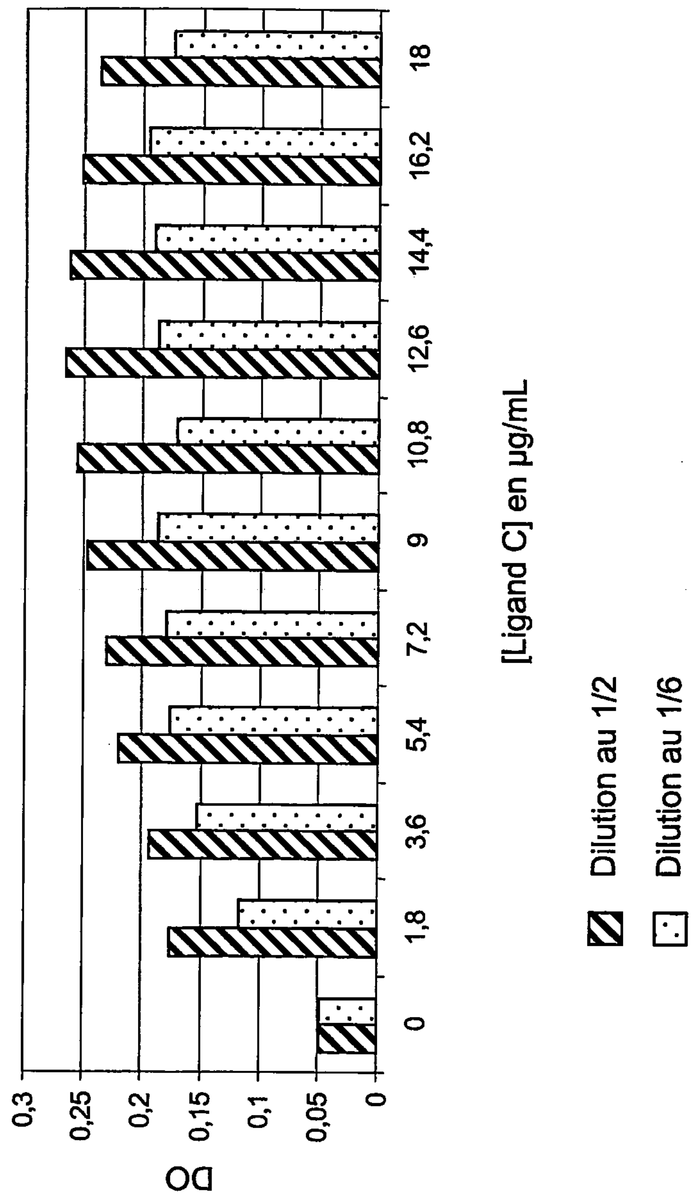
DO
0,3
0,25
0,2
0,15
0,1
0,05
0
0
1,8
3,6
5,4
7,2
9
10,8
12,6
14,4
16,2
18
[Ligand C] en µg/mL
Dilution au 1/2
Dilution au 1/6

Figure 11

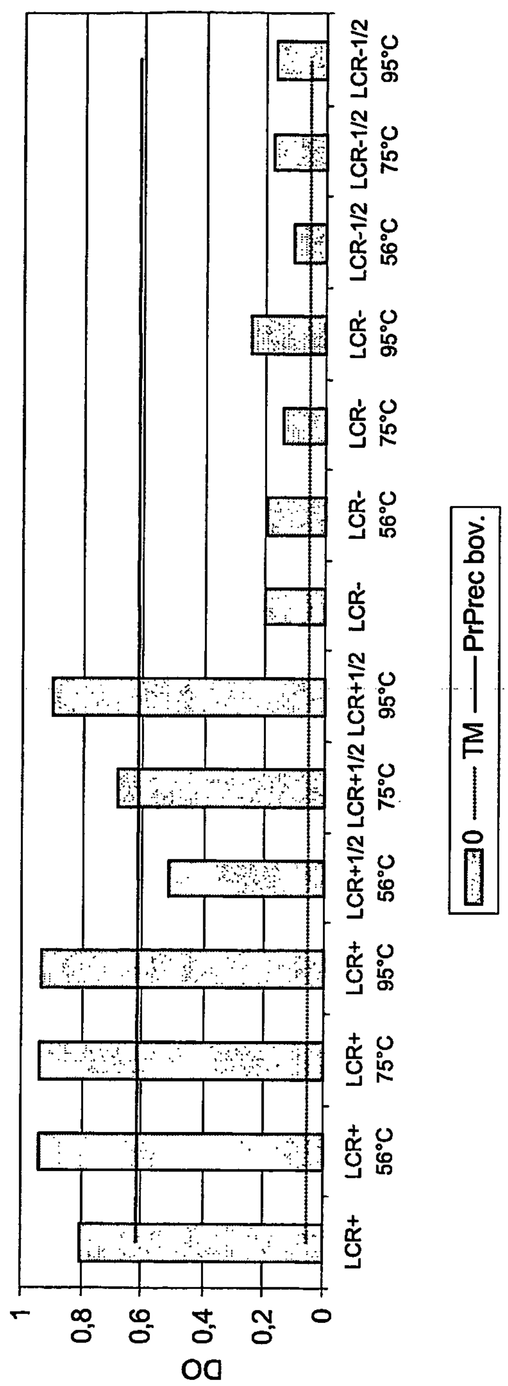
DO
1
0,8
0,6
0,4
0,2
0
LCR+
LCR+ 56°C
LCR+ 75°C
LCR+ 95°C
LCR+1/2 56°C
LCR+1/2 75°C
LCR+1/2 95°C
LCR-
LCR- 56°C
LCR- 75°C
LCR- 95°C
LCR-1/2 56°C
LCR-1/2 75°C
LCR-1/2 95°C
0
TM
PrPrec bov.

Figure 12